# EUROPEAN PATENT SPECIFICATION

(11) **EP 2 111 465 B1**
(45) Date of publication and mention of the grant of the patent: **04.12.2013**
(21) Application number: 08728023.6
(22) Date of filing: 22.01.2008
(51) Int. Cl.: C12Q 1/68

(54) **REAGENTS AND METHODS FOR DETECTING CYP2C9 POLYMORPHISMS**
REAGENZIEN UND VERFAHREN FÜR DEN NACHWEIS VON CYP2C9-POLYMORPHISMEN
RÉACTIFS ET PROCÉDÉS POUR DÉTECTER DES POLYMORPHISMES DANS CYP2C9

(30) Priority: 22.01.2007 US 881740 P
(43) Date of publication of application: 28.10.2009
(73) Proprietor: Siemens Healthcare Diagnostics Inc., Tarrytown, NY 10591 (US)
(72) Inventor: ZHENG, Minxue, Foster City, CA 94404 (US); KU, Lailing, Pleasanton, CA 94588 (US); SHEN, Lu-Ping, San Leandro, CA 94577 (US); WONG, Carrie, San Francisco, CA 94122 (US); BUSH-DONOVAN, Charlene, Livermore, CA 94551 (US)
(74) Representative: Maier, Daniel Oliver
(86) International application number: PCT/US2008/051613
(87) International publication number: WO 2008/091839

(56) References cited:
- WO-A-00/12757
- DE-A1-102004 006 477
- US-A1- 2005 272 080
- LEE CRAIG R ET AL: "Cytochrome P450 2C9 polymorphisms: A comprehensive review of the in-vitro and human data" PHARMACOGENETICS, CHAPMAN & HALL, LONDON, GB, vol. 12, no. 3, 1 April 2002 (2002-04-01), pages 251-263, XP009105154 ISSN: 0960-314X
- CYTOCHROME (CYP) P450 ALLELE NOMENCLATURE COMMITTEE: "CYP2C9 allele nomenclature" INTERNET CITATION, 11 March 2008 (2008-03-11), XP002494076

## Description

### Background of the Invention

It is well recognized that individuals exhibit considerable variability with respect to their response to pharmaceutical agents and other chemicals. Some drugs work well in some patient populations but not as well in others. Some patients experience undesirable or even toxic side effects at drug doses that would be considered appropriate for use in a typical individual, while in other cases a higher than usual dose is required for efficacy. This variability in drug response poses a significant challenge both in terms of selecting appropriate therapeutic agents and doses for the individual patient and in terms of predicting dosing, safety, and efficacy for newly developed drugs. It has been estimated that adverse drug reactions attributed to drugs that were "properly prescribed and administered" result in over 100,000 deaths annually in the United States alone (K. Lazarou et al., JAMA, 1998, 279: 1200-1205). Individual variability in drug response may well be at least in part responsible for a significant fraction of these poor outcomes as well as for the therapeutic failures that are frequently encountered in a wide range of diseases.

One of the major determinants of inter-individual variability in drug response is the existence, among individuals, of differences in genes that encode enzymes responsible for various aspects of drug metabolism. The science of pharmacogenetics encompasses the identification and analysis of differences in individual genetic makeup that influence response to drug treatment. Once a correlation between genotype and drug response has been established, information about an individual patient's genotype can be used to guide the choice of appropriate therapeutic agents and/or the selection of an appropriate dose of an agent for that patient. For example, if a patient is recognized as having a genotype associated with reduced metabolism of a particular therapeutic agent relative to metabolism of that agent in most individuals, that agent could be avoided or prescribed at a reduced dose, or the patient could be closely monitored for toxicity.

Enzymes involved in the bio-transformation of drugs are also responsible for bio-transformation of other xenobiotics, including chemicals encountered in the environment or workplace that have been linked to disease. Thus, understanding inter-individual differences in the metabolism of these compounds could help to identify individuals who may be at risk so that appropriate measures could be taken to minimize such risk. Identification and analysis of genetic polymorphisms that are associated with differences in the metabolism of drugs and other xenobiotics is thus of great interest, and considerable progress has been made in this area.

Enzymes of the cytochrome P450 family are known to play a major role in the bio-transformation of drugs and other xenobiotics as well as a variety of endogenous substances. The enzymes are predominantly found in the liver and are responsible for metabolizing more than 50% of all currently marketed drugs. Polymorphisms of the cytochrome P450 2C9 (CYP2C9) locus are a common cause of pharmacogenetic variability in humans. CYP2C9, which hydroxylates approximately 16% of therapeutic agents in current clinical use, metabolizes numerous classes of drugs including non-steroidal anti-inflammatories, oral anticoagulants (most notably warfarin), angiotensin II blockers, the alkylating anti-cancer pro-drugs, Sulfonylureas, some anti-depressants, tamoxifen, and oral hypoglycemics.

Single nucleotide polymorphisms (SNPs) in the CYP2C9 gene have increasingly been recognized as determinants of the metabolic phenotype that underlies inter-individual and ethnic differences. The normal functional allele (wild-type) of the CYP2C9 gene is designated CYP2C9*1. At least five allelic variants have been identified that lead to reduced or deficient metabolic activity. The most common poor metabolizer (PM) phenotypes have been identified as CYP2C9*2 (430 C>T) and CYP2C9*3 (1075 A>C). Over 30% of European and Caucasian populations have one or both of the CYP2C9*2 and CYP2C9*3 alleles, with allele frequencies of 0.1 and 0.008, respectively (A.K. Daly and B.P. King, Pharmacogenetics, 2003, 13: 1-6). CYP2C9*2 and CYP2C9*3 are, however, extremely rare in Asian and African American populations, with over 95% of these populations having the wild-type genotype. CYP2C9*4 (1076 T>C) has been exclusively identified in Japanese patients, and CYP2C9*5 (1080 C>G) and CYP2C9*6 (818 delA) have been found in African Americans with low allelic frequency of 0.017 and 0.006, respectively. CYP2C9*6 is a single nucleotide deletion that causes translation frame shifting, consequently the 2C9 protein product would be totally non-functional. The homozygote genotype of 2C9*6 has only been identified in one African American individual. Subjects who are carriers of one or more variant alleles may be at risk for adverse drug reactions/toxicities when prescribed drugs predominantly metabolized by CYP2C9. Individuals expressing the CYP2C9*2 and/or CYP2C9*3 genotypes also appear to be significantly more susceptible to adverse events with drugs that have narrow therapeutic indexes, such as S-warfarin, tolbutamide and phenytoin, particularly during the initiation of therapy.

A variety of methods have been developed to assess CYP2C9 enzyme activity. Many of these methods involve administration of a test compound to a subject and measurement of its metabolism by CYP2C9-mediated pathways. The discovery of genetic polymorphisms responsible for inter-individual differences in CYP2C9 has allowed the development of assays based on detection of variations in the genomic sequence of the CYP2C9 gene, such as those disclosed in DE 10 2004006477 A1. However, there remains a need for improved methods of detecting polymorphisms in CYP2C9 and for genotyping individuals with respect to their CYP2C9 alleles.

### Summary of the Invention

The invention is defined in the appended claims. The present invention is directed to systems for the rapid, reliable, and convenient detection of CYP2C9 polymorphisms of clinical significance. In particular, the present invention provides reagents and methods for the detection of polymorphisms of CYP2C9 associated with adverse drug response. More specifically, the present invention provides CYP2C9-specific oligonucleotide sequences for amplification primers and detection probes that can be used to detect CYP2C9 single nucleotide polymorphisms (SNPs). In certain embodiments, the inventive oligonucleotide sequences are useful for the detection of CYP2C9(*2), CYP2C9(*3), CYP2C9(*4), and CYP2C9(*5) alleles.

In one aspect, the present disclosure provides isolated oligonucleotides comprising a nucleic acid sequence selected from the group consisting of SEQ. ID NOs. 1-36, complementary sequences thereof, active fragments thereof, and combinations thereof. In particular, the present invention provides isolated oligonucleotide amplification primers comprising a nucleic acid sequence selected from the group consisting of SEQ. ID NOs. 1-4, active fragments thereof, and combinations thereof; isolated oligonucleotide detection probes comprising a nucleic acid sequence selected from the group consisting of SEQ. ID NOs. 5-12, SEQ. ID NOs. 29-36, active fragments thereof, and combinations thereof; and isolated oligonucleotide universal probes comprising a nucleic acid sequence selected from the group consisting of SEQ. ID NOs. 13-28, active fragments thereof, and combinations thereof.

In another aspect the present disclosure provides primer pairs, probe pairs, and primer/probe pairs that can be used for the amplification and/or detection of CYP2C9 SNPs.

In particular, the present disclosure provides a primer pair for amplifying a portion of CYP2C9 genomic sequence by PCR, wherein the primer pair is selected from the group consisting of: a primer pair which, when used in the PCR reaction, generates an amplification product that encompasses nucleotides 258247 to 258556 of the CYP2C9 genomic sequence; and a primer pair which, when used in the PCR reaction, generates an amplification product that encompasses nucleotides 297373 to 297612 of the CYP2C9 genomic sequence. Examples of such inventive primer pairs include Primer Pair 1 which comprises a forward primer comprising SEQ. ID NO. 1 or any active fragment thereof, and a reverse primer comprising SEQ ID NO. 2 or any active fragment thereof; and Primer Pair 2 which comprises a forward primer comprising SEQ. ID NO. 3 or any active fragment thereof, and a reverse primer comprising SEQ. ID NO. 4.

The present disclosure also provides pairs of allele-specific extension probes which can distinguish between CYP2C9 alleles that differ at a polymorphic position when used in a primer extension reaction, wherein the first of each pair of extension probes is complementary to a wild-type CYP2C9 allele at a polymorphic position and the second of said pair of extension probes is complementary to a mutant CYP2C9 allele at the polymorphic position, wherein said polymorphic position is selected from the group consisting of nucleotide 430, nucleotide 1075, nucleotide 1076, and nucleotide 1080. Examples of such inventive pairs of allele-specific extension probes include Probe Pair 1 which comprises a wild-type probe comprising SEQ ID NO. 5 or any active fragment thereof, and a mutant probe comprising SEQ ID NO. 6 or any active fragment thereof; Probe Pair 2(*3) which comprises a wild-type probe comprising SEQ ID NO. 7 or any active fragment thereof, and a mutant probe comprising SEQ ID NO. 8 or any active fragment thereof; Probe Pair 2(*4) which comprises a wild-type probe comprising SEQ ID NO. 9 or any active fragment thereof, and a mutant probe comprising SEQ ID NO. 10 or any active fragment thereof; and Probe Pair 2(*5) which comprises a wild-type probe comprising SEQ. ID NO. 11 or any active fragment thereof, and a mutant probe comprising SEQ. ID NO. 12.

In certain embodiments, the wild-type probe of an inventive pair of allele-specific extension probes comprises a first universal tag sequence attached at its 5' end, and the mutant probe of said pair of allele-specific extension probes comprises a second universal tag sequence attached at its 5' end, wherein the first and second tag sequences are different and selected from the group consisting of SEQ ID NOs. 13-20. Examples of such inventive pairs of allele-specific extension probes include Probe Pair 1' which comprises a wild-type probe comprising SEQ. ID NO. 29 or any active fragment thereof, and a mutant probe comprising SEQ ID NO. 30 or any active fragment thereof; Probe Pair 2'(*3) which comprises a wild-type probe comprising SEQ. ID NO. 31 or any active fragment thereof, and a mutant probe comprising SEQ ID NO. 32 or any active fragment thereof; Probe Pair 2'(*4) which comprises a wild-type probe comprising SEQ. ID NO. 33 or any active fragment thereof, and a mutant probe comprising SEQ ID NO. 34 or any active fragment thereof; and Probe Pair 2'(*5) which comprises a wild-type probe comprising SEQ. ID NO. 35 or any active fragment thereof, and a mutant probe comprising SEQ. ID NO. 36.

The present disclosure further provides primer/probe sets for detecting a CYP2C9 single nucleotide polymorphism. Examples of inventive primer/probe sets include (a) Primer Pair 1 which comprises a forward primer comprising SEQ. ID NO. 1 or any active fragment thereof, and a reverse primer comprising SEQ ID NO. 2 or any active fragment thereof, and Probe Pair 1 which comprises a wild-type probe comprising SEQ ID NO. 5 or any active fragment thereof, and a mutant probe comprising SEQ ID NO. 6 or any active fragment thereof; and (b) Primer Pair 2 which comprises a forward primer comprising SEQ. ID NO. 3 or any active fragment thereof, and a reverse primer comprising SEQ. ID NO. 4, and at least one probe pair selected from the group consisting of: Probe Pair 2(*3) which comprises a wild-type probe comprising SEQ ID NO. 7 or any active fragment thereof, and a mutant probe comprising SEQ ID NO. 8 or any active fragment thereof; Probe Pair 2(*4) which comprises a wild-type probe comprising SEQ ID NO. 9 or any active fragment thereof, and a mutant probe comprising SEQ ID NO. 10 or any active fragment thereof; and Probe Pair 2(*5) which comprises a wild-type probe comprising SEQ. ID NO. 11 or any active fragment thereof, and a mutant probe comprising SEQ. ID NO. 12.

Other examples of inventive primer/probe sets include: (a) Primer Pair 1 which comprises a forward primer comprising SEQ. ID NO. 1 or any active fragment thereof, and a reverse primer comprising SEQ ID NO. 2 or any active fragment thereof, and Probe Pair 1' which comprises a wild-type probe comprising SEQ. ID NO. 29 or any active fragment thereof, and a mutant probe comprising SEQ ID NO. 30 or any active fragment thereof; and (b) Primer Pair 2 which comprises a forward primer comprising SEQ. ID NO. 3 or any active fragment thereof, and a reverse primer comprising SEQ. ID NO. 4, and at least one probe pair selected from the group consisting of: Probe Pair 2'(*3) which comprises a wild-type probe comprising SEQ. ID NO. 31 or any active fragment thereof, and a mutant probe comprising SEQ ID NO. 32 or any active fragment thereof; Probe Pair 2'(*4) which comprises a wild-type probe comprising SEQ. ID NO. 33 or any active fragment thereof, and a mutant probe comprising SEQ ID NO. 34 or any active fragment thereof; and Probe Pair 2'(*5) which comprises a wild-type probe comprising SEQ. ID NO. 35 or any active fragment thereof, and a mutant probe comprising SEQ. ID NO. 36.

The present invention provides a kit comprising a collection of primer pairs, wherein said primer pairs are suitable for use in a single-plex or multiplex PCR reaction that comprises human genomic DNA, said collection of primer pairs comprising: a primer pair which, when used in the PCR reaction, generates an amplification product that encompasses nucleotides 258247 to 258556 of the CYP2C9 genomic sequence; and a primer pair which, when used in the PCR reaction, generates an amplification product that encompasses nucleotides 297373 to 297612 of the CYP2C9 genomic sequence.

In certain embodiments, the primer pairs do not significantly amplify CYP2C19 genomic sequences present in the PCR reaction. In certain embodiments, the collection of primer pairs comprises Primer Pair 1 and Primer Pair 2, as described above.

The kit further comprises a collection of allele-specific extension probe pairs, wherein said probe pairs can distinguish between CYP2C9 alleles that differ at a polymorphic position when used in a primer extension reaction, wherein the first of said extension probes is complementary to a wild-type CYP2C9 allele at the polymorphic position and the second of said extension probes is complementary to a mutant CYP2C9 allele at the polymorphic position, wherein said polymorphic position is selected from the group consisting of nucleotide 430, nucleotide 1075, nucleotide 1076, and nucleotide 1080. For example, the collection of allele-specific extension probe pairs may comprise Probe Pair 1 and at least one of: Probe Pair 2(*3), Probe Pair 2(*4), and Probe Pair 2(*5), as described above. The probes may be attached to a solid support, e.g., microparticles or array. Alternatively, the collection of allele-specific extension probes may comprise Probe Pair 1', and at least one of: Probe Pair 2'(*3), Probe Pair 2'(*4), and Probe Pair 2'(*5), as described above. In such embodiments, the kit may further comprise at least one zipcode sequence attached to a solid support (e.g., microparticles or array), wherein the zipcode sequence comprises a sequence selected from the group consisting of SEQ. ID NOs. 21-28.

In still another aspect, the present disclosure provides a method for determining which of a plurality of polymorphic variants of a CYP2C9 polymorphic site is present in an individual, the method comprising steps of: (a) providing a sample containing genomic DNA obtained from the individual; (b) contacting the sample with at least one allele-specific extension probe, wherein said extension probe comprises a portion that hybridizes to a target sequence of CYP2C9 genomic sequence immediately adjacent to a polymorphic position and that has a 3' terminal nucleotide that is complementary to the nucleotide at said polymorphic position so that said extension probe hybridizes to a CYP2C9 polymorphic variant that contains, at said polymorphic position, a nucleotide complementary to the 3' terminal nucleotide of said extension probe to form a hybrid; (c) subjecting the hybrid to conditions suitable for extension to form an extension product; and (d) detecting the extension product, wherein detection of the extension product is indicative of the identity of one particular polymorphic variant of the CYP2C9 polymorphic site.

In certain embodiments, the CYP2C9 polymorphic site is selected from the group consisting of: CYP2C9(*2), CYP2C9(*3), CYP2C9(*4), and CYP2C9(*5); and the extension probe comprises a sequence selected from the group consisting of SEQ. ID NOs. 5-12, SEQ. ID NOs. 29-36, active fragments thereof, and combinations thereof.

In certain embodiments, the step of contacting comprises contacting the sample with at least one pair of allele-specific probes, wherein said pair of allele-specific extension probes comprises comprising a first extension probe comprising a portion that hybridizes to a target sequence of CYP2C9 genomic sequence immediately adjacent to a polymorphic position and that has a 3' terminal nucleotide that is complementary to a non-mutated/wild-type base at said polymorphic position, and a second extension probe comprising a portion that hybridizes to a target sequence of CYP2C9 genomic sequence immediately adjacent to said polymorphic position and that has a 3' terminal nucleotide that is complementary to a mutated/mutant base at said polymorphic position. In such embodiments, the step of detecting the extension product may comprise identifying the polymorphic variant as wild-type if the extension product results from the extension of the first extension probe and identifying the polymorphic variant as mutant if the extension product results from the extension of the second extension probe.

The pair of allele-specific probes used in such methods may be selected from the group consisting of Probe Pair 1, Probe Pair 2(*3), Probe Pair 2(*4), and Probe Pair 2(*5), as described above.

In certain embodiments, the wild-type probe of the pair of allele-specific extension probes comprises a first universal tag sequence attached at its 5' end, and the mutant probe of said pair comprises a second universal tag sequence attached at its 5' end, wherein the first and second tag sequences are different and selected from the group consisting of SEQ ID NOs. 13-20. Examples of such pairs of allele-specific extension probes include Probe Pair 1', Probe Pair 2'(*3), Probe Pair 2'(*4), and Probe Pair 2'(*5), as described above. In such embodiments, the step of detecting the extension product may comprise contacting the sample comprising the extension product with at least two zipcode sequences attached to a solid support (e.g., microbeads or array), wherein the first zipcode sequence is complementary to the first universal tag sequence attached to the first and the second zipcode sequence is complementary to the second universal tag sequence. For example, the first and second zipcode sequences may comprise SEQ ID NO. 21 and SEQ ID NO. 22, respectively if the pair of allele-specific extension probes is Probe Pair 1'; SEQ ID NO. 23 and SEQ ID NO. 24 if the pair of allele-specific extension probes is Probe Pair 2'(*3); SEQ ID NO. 25 and SEQ ID NO. 26 if the pair of allele-specific extension probes is Probe Pair 2'(*4); and SEQ ID NO. 27 and SEQ ID NO. 28 if the pair of allele-specific extension probes is Probe Pair 2'(*5).

In certain embodiments, the method further comprises a step of submitting the sample to amplification prior to the contacting step. For example, said amplification may be performed using at least one primer comprising a sequence selected from the group consisting of SEQ ID NOs. 1-4, active fragments thereof, and combinations thereof. In particular, said amplification may be performed by PCR using at least one primer pair selected from the group consisting of: Primer Pair 1 and Primer Pair 2, as described above.

In certain embodiments, the method further comprises a step of selecting a therapeutic regimen for the individual, based on the identity of the polymorphic variant at the CYP2C9 polymorphic site.

These and other objects, advantages and features of the present invention will become apparent to those of ordinary skill in the art having read the following detailed description of the preferred embodiments.

### Brief Description of the Drawing

**Table 1** shows examples of inventive specific amplification primer sequences that can be used in either single amplification or multiplex amplification of the desired fragments of CYP2C9 genomic sequence. Sequences are listed from 5' to 3' direction.

**Table 2** shows examples of inventive detection probe sequences that can be used in either single amplification or multiplex detection of mutations on the CYP2C9 genomic sequence.

**Table 3** shows examples of inventive 25-mer universal tag sequences (R) that are reverse complement to orthogonal zipcode sequences (NC).

**Table 4** shows examples of inventive detection probes, each labeled with an inventive universal tag sequence. Such detection probes can be used in ASPE reactions for the detection and/or identification of CYP2C9 SNPs.

**Figure 1** presents agarose gel electrophoretic profiles of PCR amplification products, p2 (310 pb) and p3 (240 pb), that include CYP2C9 SNP sites of interest, that were obtained as described in Example 1 using amplification primers of the present invention.

**Figure 2** presents results of CYP2C9 assay genotype of a single patient based on data obtained using the Luminex-100 system. As shown on this figure, the individual tested was found to be heterozygous for 1075 A>C, and wild-type for other polymorphisms.

**Figure 3** presents results of an assay carried out to test the specificity of the inventive universal capture sequences. Data were recorded using the Luminex-100 system.

**Figure 4** presents the genotype results of a panel of seven patient samples that include all available genotypes. The results, which are presented in scattered plots for each allele, were obtained using the Luminex system.

**Figure 5** presents the genotype results of a single patient sample, which were obtained using a planar waveguide (PWG) technique. In this figure, the SNPs are identified/numbered using the nucleotide changes in the gene rather than the nucleotide changes in cDNA. Correspondences between the cDNA identification/numbering system and the gene identification/numbering system are as follows: 430 C>T (in cDNA) corresponds to 3608 C>T (in gene); 1075 A>C corresponds to 42614 A>C; 1076 T>C corresponds to 42615 T>C; and 1080 C>G corresponds to 42619 C>G.

### Definitions

Throughout the specification, several terms are employed that are defined in the following paragraphs.

The term "*gene*", as used herein, has its art understood meaning, and refers to a part of the genome specifying a macromolecular product, be it a functional RNA molecule or a protein, and may include regulatory sequences (*e.g*., promoters, enhancers, etc) and/or intro sequences preceding (5' non-coding sequences) and following (3' non-coding sequences) the coding sequences. For example, as used herein, the CYP2C9 gene includes the CYP2C9 promoter region, as well as a non-coding nucleic acid sequence that is present in the CYP2C9 transcript (*e.g*., 5' and/or 3' unstranslated regions).

A **"*gene product*"** or **"*expression product*"** is an RNA transcribed from the gene (e.g., either pre- or post-processing) and/or a polypeptide encoded by an RNA transcribed from the gene (e.g., either pre- or post-modification). RNA transcribed from a gene or polynucleotide is said to be encoded by the gene or polynucleotide. Similarly, a polypeptide generated by translation of a messenger RNA is said to be encoded by that messenger RNA, and is also said to be encoded by the gene from which the messenger RNA is transcribed.

As used herein, the term **"*wild-type*"** refers to a gene, gene portion or gene product that has the characteristics of that gene, gene portion or gene product when isolated from a naturally-occurring source. A wild-type gene has the sequence that is the most frequently observed in a population and is thus arbitrarily designated as the **"*normal*"** or **"*wild-type*"** sequence.

The terms **"*allele*"** and **"*allelic variants*"** are used herein interchangeably. They refer to alternative forms of a gene or a gene portion. Alleles occupy the same locus or portion on homologous chromosomes. When an individual has two identical alleles of a gene, the individual is said to be homozygous for the gene or allele. When an individual has two different alleles of a gene, the individual is said to be heterozygous for the gene. Alleles of a specific gene can differ from each other in a single nucleotide or a plurality of nucleotides, and can include substitutions, deletions and/or insertions of nucleotides with respect to each other. An allele of a gene can also be a form of a gene containing a mutation. While the terms "allele" and "allelic variant" have traditionally been applied in the context of genes, which can include a plurality of polymorphic sites, the term may also be applied to any form of a genomic DNA sequence, which may or may not fall within a gene. Thus each polymorphic variant of a polymorphic site can be considered as an allele of that site. The term **"*allele frequency*"** refers to the frequency at which a particular polymorphic variant, or allele, occurs in a population being tested (*e.g*., between cases and controls in an association study).

The term **"*polymorphism*"** refers to the occurrence of two or more alternative genomic DNA sequences or alleles that exist and are inherited within a population. Either of the sequences themselves, or the site at which they occur, may also be referred to as a polymorphism. If a polymorphism is located within a portion of the genome that is transcribed into RNA, the collective RNA of that population will also contain a polymorphism at that position. A **"*single nucleotide polymorphism*** or ***SNP*"** is a polymorphism that exists at a single nucleotide position. A **"*polymorphic site*", "*polymorphic position"*** or **"*polymorphic locus*"** is a location at which differences in genomic DNA exist among members of a population. While in general the polymorphic sites of interest in the context of the present invention are single nucleotides, the term is not limited to sites that are only one nucleotide in length. A **"*polymorphic region*"** is a region of genomic DNA that includes one or more polymorphic sites.

The term **"*polymorphic variant*"** refers to any of the alternate sequences that may exist at a polymorphic site among members of a population. For purpose of the present invention, the population may be the population of the world, or a subset thereof. For the methods described herein, it will typically be of interest to determine which polymorphic variant(s) (as among multiple polymorphic variants that exist within a population) is/are present in an individual.

As used herein, the term **"*genotype*"** refers to the identity of an allelic variant at a particular polymorphic position in an individual. It will be appreciated that an individual's genome will contain two allelic variants for each polymorphic position (located on homologous chromosomes). The allelic variants can be the same or different. A genotype can include the identity of either or both alleles. A genotype can include the identities of allelic variants at multiple different polymorphic positions, which may or may not be located within a single gene. A genotype can also refer to the identity of an allele of a gene at a particular gene locus in an individual and can include the identity of either or both alleles. The identity of the allele of a gene may include the identity of the polymorphic variants that exist at multiple polymorphic sites within the gene. The identity of an allelic variant or an allele of a gene refers to the sequence of the allelic variant or allele of a gene (*e.g.*, the identity of the nucleotide present at a polymorphic position or the identities of nucleotides present at each of the polymorphic positions in a gene). It will be appreciated that the identity need not be provided in terms of the sequence itself. For example, it is typical to assign identifiers such as +, -, A, a, B, b, etc to different allelic variants or alleles for descriptive purposes. Any suitable identifier can be used. **"*Genotyping*"** an individual refers to providing the genotype of the individual with respect to one or more allelic variants or alleles.

The terms **"*individual*"** and **"*subject*"** are used herein interchangeably. They refer to a human being. The terms do not denote a particular age, and thus encompass adults, children, newborns, as well as fetuses.

As used herein, a **"*sample*"** obtained from an individual may include, but is not limited to, any or all of the following: a cell or cells, a portion of tissue, blood, serum, ascites, urine, saliva, amniotic fluid, cerebrospinal fluid, and other body fluids, secretion, or excretions. The sample may be a tissue sample obtained, for example, from skin, muscle, buccal or conjunctival mucosa, placenta, gastrointestinal tract or other organs. A sample of DNA from fetal or embryonic cells or tissue can be obtained by appropriate methods, such as by amniocentesis or chorionic villus sampling. Samples may also include sections of tissues such as frozen sections. The term "sample" also includes any material derived by isolating, purifying, and/or processing a sample as previously defined. Derived materials include, but are not limited to, cells (or their progeny) isolated from the sample, cell components, nucleic acids or proteins extracted from the sample or obtained by subjecting the sample to techniques such as amplification or reverse transcription of mRNA, etc. Processing of the sample may involve one or more of: filtration, distillation, centrifugation, extraction, concentration, dilution, purification, inactivation of interfering components, addition of reagents, and the like.

The terms **"*genomic DNA*"** and **"*genomic nucleic acid*"** are used herein interchangeably. They refer to nucleic acid from the nucleus of one or more cells, and include nucleic acid derived from (*e.g*., isolated from, cloned from) genomic DNA. The terms **"*sample of genomic DNA*"** and **"*sample of genomic nucleic acid*"** are used herein interchangeably and refer to a sample comprising DNA or nucleic acid representative of genomic DNA isolated from a natural source and in a form suitable for hybridization to another nucleic acid (*e.g.*, as a soluble aqueous solution). Samples of genomic DNA to be used in the practice of the present invention generally include a plurality of nucleic acid segments (or fragments) which together may cover a substantially complete genome or a portion of the genome comprising the CYP2C9 gene. A sample of genomic DNA may be isolated, extracted or derived from solid tissues, body fluids, skeletal tissues, or individual cells. A sample of genomic DNA can be isolated, extracted or derived from fetal or embryonic cells or tissues obtained by appropriate methods, such as amniocentesis or chronic villus sampling.

The terms ***"nucleic acid*"*,* "*nucleic acid molecule*",** and **"*polynucleotide*"** are used herein interchangeably. They refer to linear polymers of nucleotide monomers or analogs thereof, such as deoxyribonucleic acid (DNA) and ribonucleic acid (RNA). Unless otherwise stated, the terms encompass nucleic acid-like structures with synthetic backbones, as well as amplification products.

As used herein, the term **"*amplification*"** refers to a process that increases the representation of a population of specific nucleic acid sequences in a sample by producing multiple (*i.e*., at least 2) copies of the desired sequences. Methods for nucleic acid amplification are known in the art and include, but are not limited to, polymerase chain reaction (PCR) and ligase chain reaction (LCR). In a typical PCR amplification reaction, a nucleic acid sequence of interest is often amplified at least fifty thousand fold in amount over its amount in the starting sample. A **"*copy*"** or **"*amplicon*"** does not necessarily mean perfect sequence complementarity or identity to the template sequence. For example, copies can include nucleotide analogs such as deoxyinosine, intentional sequence alterations (such as sequence alterations introduced through a primer comprising a sequence that is hybridizable but not complementary to the template), and/or sequence errors that occur during amplification.

The term **"*oligonucleotide*",** as used herein, refers to a string of nucleotides or analogs thereof. These stretches of nucleic acid sequences may be obtained by a number of methods including, for example, chemical synthesis, restriction enzyme digestion or PCR. As will be appreciated by one skilled in the art, the length of an oligonucleotide (*i.e*., the number of nucleotides it contains) can vary widely, often depending on its intended function or use. Generally, oligonucleotides comprise between about 5 and about 150 nucleotides, usually between about 10 and about 100 nucleotides, and more usually between about 15 and about 75 nucleotides, or between about 15 and about 50 nucleotides. Throughout the specification, whenever an oligonucleotide is represented by a sequence of letters (chosen, for example, from the four base letters: A, C, G, and T, which denote adenosine, cytidine, guanosine, and thymidine, respectively), the nucleotides are presented in the 5'→3' order from the left to the right. In certain embodiments, the sequence of an oligonucleotide of the present invention contains the letter M and/or letter Y. As used herein, the letter "***M***" represents a degenerative base, which can be A or C with substantially equal probability. As used herein, the letter "***Y***" represents a degenerative base, which can be T or C with substantially equal probability. Thus, for example, in the context of the present invention, if an oligonucleotide contains one degenerative base M, the oligonucleotide is a substantially equimolar mixture of two subpopulations of a first oligonucleotide where the degenerative base is A and a second oligonucleotide where the degenerative base is C, the first and second oligonucleotides being otherwise identical.

The term "***3***'" refers to a region or position in a polynucleotide or oligonucleotide 3' (*i.e.*, downstream) from another region or position in the same polynucleotide or oligonucleotide. The term "**5**' " refers to a region or position in a polynucleotide or oligonucleotide 5' (i.e., upstream) from another region or position in the same polynucleotide or oligonucleotide. The terms *"**3' end***" and *"****3' terminus*",** as used herein in reference to a nucleic acid molecule, refer to the end of the nucleic acid which contains a free hydroxyl group attached to the 3' carbon of the terminal pentose sugar. The term **"*5*' *end*"** and **"*5*' *terminus",*** as used herein in reference to a nucleic acid molecule, refers to the end of the nucleic acid molecule which contains a free hydroxyl or phosphate group attached to the 5' carbon of the terminal pentose sugar.

The term **"*isolated*",** as used herein in reference to an oligonucleotide, means an oligonucleotide, which by virtue of its origin or manipulation, is separated from at least some of the components with which it is naturally associated or with which it is associated when initially obtained. By "isolated", it is alternatively or additionally meant that the oligonucleotide of interest is produced or synthesized by the hand of man.

The terms **"*target nucleic acid*"** and **"*target sequence"*** are used herein interchangeably. They refer to a nucleic acid sequence, the presence or absence of which is desired to be determined/detected. The target sequence may be single-stranded or double-stranded. If double-stranded, the target sequence may be denatured to a single-stranded form prior to its detection. This denaturation is typically performed using heat, but may alternatively be carried out using alkali, followed by neutralization. In the context of the present invention, a target sequence comprises at least one single nucleotide polymorphic site. Preferably, target sequences comprise nucleic acid sequences to which primers can hybridize, and/or probe-hybridization sequences with which probes can form stable hybrids under desired conditions.

The term **"*hybridization*",** as used herein, refers to the formation of complexes (also called duplexes or hybrids) between nucleotide sequences which are sufficiently complementary to form complexes *via* Watson-Crick base pairing or non-canonical base pairing. It will be appreciated that hybridizing sequences need not have perfect complementary to provide stable hybrids. In many situations, stable hybrids will form where fewer than about 10% of the bases are mismatches. Accordingly, as used herein, the term **"*complementary*"** refers to a nucleic acid molecule that forms a stable duplex with its complement under assay conditions, generally where there is about 90% or greater homology. Those skilled in the art understand how to estimate and adjust the stringency of hybridization conditions such that sequences that have at least a desired level of complementarity will stably hybridize, while those having lower complementarity will not. For examples of hybridization conditions and parameters, see, for example, J. Sambrook et al., "Molecular Cloning: A Laboratory Manual", 1989, Second Edition, Cold Spring Harbor Press: Plainview, NY; F.M. Ausubel, "Current Protocols in Molecular Biology", 1994, John Wiley & Sons: Secaucus, NJ. Complementarity between two nucleic acid molecules is said to be **"*complete*", "*total*"** or **"*perfect*"** if all the nucleic acids' bases are matched, and is said to be **"*partial*"** otherwise.

The term **"*melting temperature*"** or "Tm" of a specific oligonucleotide, as used herein, refers to the specific temperature at which half of the oligonucleotide hybridizes to its target in equilibrium. Accurate prediction of the Tm of any oligonucleotide can be made based on sequence using nearest neighbor parameter calculations. Tm is one of the most important parameters in the design of primers and probes for a given assay. In certain embodiments, ASPE probes described herein were designed to have higher Tm's than Tm's of the amplification primers.

The terms **"*probes*"** and **"*primers*",** as used herein, typically refer to oligonucleotides that hybridize in a sequence specific manner to a complementary nucleic acid molecule (*e.g*., a nucleic acid molecule comprising a target sequence). The term "primer", in particular, generally refers to an oligonucleotide that acts as a point of initiation of a template-directed synthesis using methods such as PCR (polymerase chain reaction) or LCR (ligase chain reaction) under appropriate conditions (*e.g*., in the presence of four different nucleotide triphosphates and a polymerization agent, such as DNA polymerase, RNA polymerase or reverse-transcriptase, DNA ligase, etc, in an appropriate buffer solution containing any necessary co-factors and at suitable temperature(s)). Such a template directed synthesis is also called "primer extension". For example, a primer pair may be designed to amplify a region of DNA using PCR. Such a pair will include a "forward primer" and a "reverse primer" that hybridize to complementary strands of a DNA molecule and that delimit a region to be synthesized/amplified.

Typically, an oligonucleotide probe or primer will comprise a region of nucleotide sequence that hybridizes to at least about 8, more preferably at least about 10 or at least about 15, typically about 20 to about 40 consecutive nucleotides of a target nucleic acid *(i.e.,* will hybridize to a contiguous sequence of the target nucleic acid). Oligonucleotides that exhibit differential or selected binding to a polymorphic site may readily be designed by one of ordinary skill in the art. For example, an oligonucleotide that is perfectly complementary to a sequence that encompasses a polymorphic site will hybridize to a nucleic acid comprising that sequence as opposed to a nucleic acid comprising an alternate polymorphic variant.

In general, a primer or probe sequence is identified as being either **"*complementary*"** (*i.e*., complementary to the coding or sense strand (+)), or **"*reverse complementary*"** (*i.e*., complementary to the anti-sense strand (-)).

The terms **"*forward primer*"** and **"*forward amplification primer*"** are used herein interchangeably, and refer to a primer that hybridizes (or anneals) to the target (template strand). The terms ***"reverse primer*"** and *"****reverse amplification primer*"** are used herein interchangeably, and refer to a primer that hybridizes (or anneals) to the complementary target strand. The forward primer hybridizes with the target sequence 5' with respect to the reverse primer.

The terms **"*probe*"** and *"****detection probe*"** are used herein interchangeably and refer to an oligonucleotide capable of selectively hybridizing to at least a portion of a target sequence under appropriate conditions. A detection probe may be labeled with a detectable moiety.

As used herein, the term **"*allele-specific primer*"** refers to a primer whose 3'-terminal base is complementary to the corresponding template base for a particular allele at the polymorphic site. An allele-specific primer may comprise a sequence that is perfectly complementary to a sequence of the template immediately upstream to the polymorphic site. The term **"*allele-specific primer extension*** or ***ASPE*"** refers to a process in which an oligonucleotide primer is annealed to a DNA template 3' with respect to a nucleotide indicative of the presence or absence of a target allele, and then extended in the presence of dNTPs (*e.g*., labeled dNTPs) and a DNA polymerase that lacks 3' nuclease activity.

The term **"*****amplification conditions"**,* as used herein, refers to conditions that promote annealing and/or extension of primer sequences. Such conditions are well-known in the art and depend on the amplification method selected. Thus, for example, in a PCR reaction, amplification conditions generally comprise thermal cycling, *i.e*., cycling of the reaction mixture between two or more temperatures. In isothermal amplification reactions, amplification occurs without thermal cycling although an initial temperature increase may be required to initiate the reaction. Amplification conditions encompass all reaction conditions including, but not limited to, temperature and temperature cycling, buffer, salt, ionic strength, and pH, and the like.

As used herein, the term **"*amplification reaction reagents",*** refers to reagents used in nucleic acid amplification reactions and may include, but are not limited to, buffers, reagents, enzymes having reverse transcriptase and/or polymerase activity or exonuclease activity, enzyme cofactors such as magnesium or manganese, salts, nicotinamide adenine dinuclease (NAD) and deoxynucleoside triphosphates (dNTPs), such as deoxyadenosine triphospate, deoxyguanosine triphosphate, deoxycytidine triphosphate and thymidine triphosphate. Amplification reaction reagents may readily be selected by one skilled in the art depending on the amplification method used.

The term **"*multiplex PCR redaction*"** refers to a PCR reaction in which multiple PCR amplifications are performed simultaneously in a single vessel or container and in which a plurality of (*i.e.,* at least 2) amplification products are generated using a plurality of primer pairs. A collection of primer pairs is suitable for use in a multiplex PCR reaction if each of the primer pairs generates a discrete amplification product under at least one set of PCR conditions, without significant interference and/or cross-reactivity by one or more members of the other primer pairs present in the multiplex PCR reaction. In certain embodiments, PCR conditions of a multiplex PCR reaction may be optimized to compensate for the particular polymerase used, particular nucleic acid sequences, polypeptides, small molecules, metabolites, inorganic ions and/or other factors present in the reaction mixture, the method or methods used to isolate the nucleic acid for amplification, and/or any of a variety of other conditions known to those of ordinary skill in the art that may affect the PCR including, but not limited to, the efficacy, fidelity, or speed of the polymerization reaction.

The term **"*active fragment*",** as used herein in reference to an oligonucleotide (e.g., an oligonucleotide sequence provided herein), refers to any nucleic acid molecule comprising a nucleotide sequence sufficiently homologous to or derived from the nucleotide sequence of the oligonucleotide, which includes fewer nucleotides than the full length oligonucleotide, and retains at least one biological property of the entire sequence. Typically, active fragments comprise a sequence with at least one activity of the full length oligonucleotide. An active fragment or portion of an oligonucleotide sequence of the present invention can be a nucleic acid molecule which is, for example, 10, 15, 20, 25, 30 or more nucleotides in length and can be used as amplification primer and/or detection probe for the detection of at least one CYP2C9 polymorphism in a sample.

The term **"*sufficiently homologous*",** when used herein in reference to an active fragment of an oligonucleotide, refers to a nucleic acid molecule that has a sequence homology of at least 35% compared to the oligonucleotide. In certain embodiments, the sequence homology is at least 40%, at least 50%, at least 60%, at least 70%, at least 80%, at least 90%, at least 95%, or more than 95%.

The terms **"*homology*"** and **"*identify*"** are used herein interchangeably, and refer to the sequence similarity between two nucleic acid molecules. Calculation of the percent homology or identity of two nucleic acid sequences can be performed by aligning the two sequences for optimal comparison purposes (*e.g*., gaps can be introduced in one or both of a first and a second nucleic acid sequences for optimal alignment and non-homologous sequences can be disregarded for comparison purposes). In certain embodiments, the length of a sequence aligned for comparison purposes is at least 30%, at least 40%, at least 50%, at least 60%, at least 70%, at least 80%, at least 90%, at least 95%, or more than 95% (*e.g*., 99%, or 100%) of the length of the reference sequence. The nucleotides at corresponding nucleotide positions are then compared. When a position in the first sequence is occupied by the same nucleotide as the corresponding position in the second sequence, then the molecules are identical (or homologous) at that position. The percent identify between the two sequences is a function of the number of identical positions shared by the sequences, taking into account the number of gaps, and the length of each gap, which needs to be introduced for optimal alignment of the two sequences.

The comparison of sequences and determination of percent identity between two sequences can be accomplished using a mathematical algorithm. For example, the percent identity between two sequences can be determined using the algorithm of Meyers and Miller (CABIOS, 1989, 4: 11-17), which has been incorporated into the ALIGN program (version 2.0), using a PAM120 weight residue table, a gap length penalty of 12 and a gap penalty of 4.

The terms **"*labeled*"** and **"*labeled with a detectable agent*** (or ***moiety*)"** are used herein interchangeably to specify that an entity (*e.g*., a target sequence) can be visualized, for example following hybridization to another entity (*e.g.,* a probe). Preferably, the detectable agent or moiety is selected such that it generates a signal which can be measured and whose intensity is related to (*e.g*., proportional to) the amount of hybrid. Methods for labeling nucleic acid molecules are well-known in the art. Labeled nucleic acids can be prepared by incorporation of, or conjugation to, a label that is directly or indirectly detectable by spectroscopic, photochemical, biochemical, immunochemical, electrical, optical, or chemical means. Suitable detectable agents, include, but are not limited to, radionuclides, fluorophores, chemiluminescent agents, microparticles, enzymes, colorimetric labels, magnetic labels, haptens, molecular beacons, and aptamer beacons.

The term **"*fluorophore*", "*fluorescent moiety*",** and **"*fluorescent dye*"** are used herein interchangeably. They refer to a molecule that absorbs a quantum of electromagnetic radiation at one wavelength, and emits one or more photons at a different, typically longer wavelength in response. Numerous fluorescent dyes of a wide variety of structures and characteristics are suitable for use in the practice of the present invention. Methods and materials are known for fluorescently labeling nucleic acid molecules (see, for example, R.P. Haugland, "Molecular" Probes: Handbook of Fluorescent Probes and Research Chemicals 1992-1994", 5th Ed., 1994, Molecular Probes, Inc.). Rather than being directly detectable themselves, some fluorescent dyes transfer energy to another fluorescent dye in a process of non-radiative fluorescence resonance energy transfer (FRET), and the second dye produces the detected signal. Such FRET fluorescent dye pairs are also encompassed by the term "fluorescent moiety". The use of physically linked fluorescent reporter/quencher molecule is also within the scope of the invention. In these embodiments, when the reporter and quencher moieties are held in close proximity, such as at the ends of a nucleic acid probe, the quencher moiety prevents detection of a fluorescent signal from the reporter moiety. When the two moieties are physically separated, for example in the absence of target, the fluorescence signal from the reporter moiety becomes detectable.

As used herein, the term **"*diagnostic information*"** refers to any information that is useful in determining whether a patient is suffering from or is susceptible to develop a disease or condition and/or in classifying the disease or condition into a phenotypic category or any category having significance with regards to the prognostic or severity of, or likely response to treatment (either treatment in general or any particular treatment), of the disease or condition. Diagnostic information can include, for example, an assessment of the likelihood that an individual will suffer an adverse drug reaction if treated with a typical dose of a particular drug. Diagnostic information includes any information useful in selecting an appropriate regimen, *e.g*., drug, drug dose, dosing interval, etc. In the context of the present invention, **"*diagnosis*"** refers to providing any type of diagnostic information, including, but not limited to, whether a subject has a particular CYP2C9 allele, whether a subject is an extensive, poor, intermediate, or ultra-rapid metabolizer of drugs that are, at least in part, metabolized by CYP2C9, whether a subject is at increased risk of suffering an adverse drug reaction relative to an individual having a "wild-type" CYP2C9 genotype, or whether a subject is at increased risk of developing a particular disease relative to an individual having a "wild-type" CYP2C9 genotype.

The term **"*microparticles*"** is used herein to refer to particles having a smallest cross-sectional dimension of 50 microns or less. For example, the smallest cross-sectional dimension may be approximately 10 microns or less, approximately 3 microns or less, approximately 1 micron or less, or approximately 0.5 microns or less, *e.g*., approximately 0.1, 0.2, 0.3 or 0.4 microns. Microparticles may be made of a variety of inorganic or organic materials including, but not limited to, glass (*e.g*., controlled pore glass), silica, zirconia, cross-linked polystyrene, polyacrylate, polymethylmethacrylate, titanium dioxide, latex, polystyrene, etc. See, for example, U.S. Pat. No. 6,406,848 for various suitable materials and other considerations. Magnetically responsive microparticles can be used. Dyna beads, available from Dynal (Oslo, Norway), are an example of commercially available microparticles suitable for use in the present invention. In certain embodiments, one or more populations of fluorescent microparticles are employed. The populations may have different fluorescence characteristics so that they can be distinguished from one another, *e.g*., using flow cytometry. Microparticles can be modified, *e.g*., an oligonucleotide may be attached to a microparticle to serve as a "zip code" that allows specific hybridization to a second oligonucleotide that comprises a portion that is complementary to the zip code as described in more detail elsewhere herein.

As used herein, the term **"*****planar** waveguide* or ***PWG*"** has its art understood meaning and refers to a spatially inhomogeneous structure with a planar geometry, which restricts the spatial region in which light can propagate. In many embodiments of the present invention, planar waveguides are in the form of chips that can be coupled to a laser. Such systems allow for the selective excitation of molecules that are in close proximity to the surface of the PWG. Thus, for example, labeled molecules (*e.g*., labeled amplicons) that are in close proximity to (*e.g*., bound to) the surface of the PWG are excited by the laser light and can be detected (*e.g*., using a CCD camera), while unbound molecules are not excited by the laser light. Such systems exhibit higher specificity in detection then more conventional chip systems.

### Detailed Description of Certain Preferred Embodiments

As mentioned above, the present invention relates to kits for selectively detecting the presence of allelic variants of cytochrome P450 CYP2C9 gene and determining their identity. In certain embodiments, the inventive methods use CYP2C9-specific oligonucleotide sequences and sensitive nucleic acid amplification-based techniques that allow for the detection of clinically relevant CYP2C9 polymorphisms, in particular, CYP2C9 polymorphisms associated with response to drugs or other xenobiotic compounds.

### I - Oligonucleotide Sequences for Amplification Primers and Detection Probes

### Inventive Oligonucleotide Sequences

The present invention provides oligonucleotide sequences that are specific for the CYP2C9 gene and do not significantly amplify the CYP2C19 gene (which is highly homologous to the gene sequence of the CYP2C9 gene). The inventive oligonucleotide sequences can be used as amplification primers and detection probes to selectively detect and identify CYP2C9 single nucleotide polymorphisms (SNPs).

In particular, oligonucleotide sequences are provided that can be used to amplify portions of the CYP2C9 genomic sequence that contain SNPs of interest. In certain embodiments, SNPs of interest are selected from the group consisting of SNPs *2 (430 C>T), 3* (1075 A>C), 4* (1076 T>C), and 5* (1080 C>G). More specifically, oligonucleotide sequences of the invention can be used to amplify a target sequence that encompasses nucleotides 258247 to 258556 of the CYP2C9 genomic sequence. Products of this PCR amplification reaction are called Amplicon p2 (310 basepairs). Other oligonucleotide sequences of this invention can be used to amplify a target sequence that encompasses nucleotides 297373 to 297612 of the CYP2C9 genomic sequence. Products of this amplification reaction are herein called Amplicon p3 (240 basepairs).

Exemplary CYP2C9-specific oligonucleotide sequences for amplification primers provided by the present invention are presented in Table 1. In particular, Amplicon p2 (as described above) can be produced by PCR using a forward primer comprising SEQ ID NO. 1 and a reverse primer comprising SEQ ID NO. 2. Amplicon p3 can be produced by PCR using a forward primer comprising SEQ ID NO. 3 and a reverse primer comprising SEQ ID NO. 4. Amplification primers were designed to exhibit similar Tm's so that comparable amplification efficient is achieved for both amplicons. Specific Tm values depend on solution conditions (including salt and target concentrations) as well as primer concentrations.

The present invention further provides oligonucleotide sequences that can be used as detection probes to detect and identify different SNPs located within Amplicon p2 *(i.e.,* SNP *2) and Amplicon p3 (*i.e.*, SNPs *3, *4, and *5).

Exemplary oligonucleotide sequences of the present invention that can be used as probes for the detection of CYP2C9 SNP *2, *3, *4, and/or *5 are presented in Table 2. Alternatively or additionally, these nucleic acid sequences can be used to identify additional CYP2C9 SNPs of clinical interest that reside within Amplicon p2 and/or Amplicon p3.

In particular, wild-type (WT) and mutant (MT) probes comprising SEQ ID. NOs. 5-12 can be used in Allele Specific Primer Extension (ASPE) reactions to specifically detect SNPs within Amplicon p2 and Amplicon p3.

More specifically, a wild-type probe comprising SEQ ID NO. 5 and a mutant probe comprising SEQ ID NO. 6 can be used to detect SNP *2. A wild-type probe comprising SEQ ID NO. 7 and a mutant probe comprising SEQ ID NO. 8 can be used to detect SNP *3. A wild-type probe comprising SEQ ID NO. 9 and a mutant probe comprising SEQ ID NO. 10 can be used to detect SNP *4. A wild-type probe comprising SEQ ID NO. 11 and a mutant probe comprising SEQ ID NO. 12 can be used to detect SNP *5. All probes were designed to have Tm's that are similar and that are higher than the Tm's of the amplification primers. The difference in Tm's between the probes and primers eliminate possible side PCR reactions between the ASPE probes and any existing reverse primers during the ASPE cycles, thus eliminating the need for a cleaning step after PCR amplification.

The close proximity of mutation sites of SNPs *3, *4 and *5 (at nucleotides 1075, 1076, and 1080 on the cDNA, respectively) creates a challenge for simultaneous detection of all three alleles. For example, due to the respective positions of these SNPs, probes for the detection of SNP *5 will necessarily contain both mutation sites of SNPs *3 and *4, which could lead to non-specific hybridization (and therefore erroneous SNP identification) if the individual tested happens to have *3 or *4 or both *3 and *4 alleles. Similarly, probes for the detection of SNP *4 will necessarily contain the mutation site of SNP *3. The present invention uses degenerate probes to solve this problem.

Thus, both wild-type and mutant probes for the detection of SNP *4 according to the present invention contain a degenerate nucleotide M (substantially equal amount of nucleotide A and nucleotide C) at the position of the *3 allele (*i.e*., nucleotide 1075). More specifically, each probe for the detection of SNP *4 (*i.e*., wild-type probe and mutant probe) is a substantially equimolar mixture of two probes, wherein the first one contains nucleotide A at the *3 mutation site and the second one contains nucleotide C at the *3 mutation site. Similarly, both wild-type and mutant probes for the detection of SNP *5 according to the present invention contain a degenerate nucleotide M (substantially equal amount of nucleotide A and nucleotide C) at the position of the *3 allele (*i.e*., nucleotide 1075) and a degenerate nucleotide Y (substantially equal amount of nucleotide T and nucleotide C) at the position of the *4 allele (*i.e*., nucleotide 1076). More specifically, each probe for the detection of SNP *5 *(i.e.,* wild-type probe and mutant probe) is a substantially equimolar mixture of four probes, wherein the first one contains nucleotide A at the *3 mutation site and nucleotide T at the *4 mutation site, the second one contains nucleotide A the *3 mutation site and nucleotide C at the *4 mutation site, the third one contains nucleotide C at the *3 mutation site and nucleotide T at the *4 mutation site, and the fourth one contains nucleotide C at the *3 mutation site and nucleotide C at the *4 mutation site.

As will be appreciated by one skilled in the art, some of the oligonucleotide sequences of the present invention may be employed either as amplification primers or detection probes depending on the intended use or assay format. For example, an inventive oligonucleotide sequence used as an amplification primer in one assay can be used as a detection probe in a different assay. A given sequence may be modified, for example, by attaching to the inventive oligonucleotide sequence, a specialized sequence (*e.g*., a promoter sequence) required by the selected amplification method, or by attaching a fluorescent dye to facilitate detection. It is also understood that an oligonucleotide according to the present invention may include one or more sequences which serve as spacers, linkers, sequences of labeling or binding to an enzyme, which may impart added stability or susceptibility to degradation process or other desirable property to the oligonucleotide.

Based on the oligonucleotide sequences provided herein, one or more oligonucleotide analogues can be prepared (see below). Such analogues may contain alternative structures such as peptide nucleic acids or "PNAs" (*i.e.*, molecules with a peptide-like backbone instead of the phosphate sugar backbone of naturally-occurring nucleic acids) and the like. These alternative structures, representing the sequences of the present invention, are likewise part of the present invention. Similarly, it is understood that oligonucleotide consisting of the sequences of the present invention may contain deletions, additions, and/or substitutions of nucleic acid bases, to the extent that such alterations do not negatively affect the properties of the nucleic acid molecules. In particular, the alterations should not result in significant lowering of the hybridizing properties of the oligonucleotides.

### Primer Sets and Primer/Probe Sets

Primers and/or probes of the present invention may be conveniently provided in sets, *e.g.*, sets that can be used to determine which polymorphic variant(s) is/are present among some or all of the possible polymorphic variants that may exist at a particular polymorphic site. Multiple sets of primers and/or probes, capable of detecting polymorphic variants at a plurality of polymorphic sites are provided.

As used herein, the term **"*primer set*"** refers to two or more primers which together are capable of priming the amplification of a nucleotide sequence of interest (*e.g*., to generate Amplicon p2 or Amplicon p3). In certain embodiments, the term "primer set" refers to a pair of primers including a 5' (upstream) primer (or forward primer) that hybridizes with the 5'-end of the nucleic acid sequence to be amplified and a 3' (downstream) primer (or reverse primer) that hybridizes with the complement of the sequence to be amplified. Such primer set or primer pair are particularly useful in PCR amplification reactions.

Examples of primer sets/pairs comprising a forward amplification primer and a reverse amplification primer include: Primer Set 1, which comprises a forward primer comprising SEQ ID NO. 1, or any active fragment thereof, and a reverse primer comprising SEQ ID NO. 2, or any active fragment thereof; and Primer Set 2, which comprises a forward primer comprising SEQ ID NO. 3, or any active fragment thereof, and a reverse primer comprising SEQ ID NO. 4, or any active fragment thereof.

In addition to primer sets, the present invention provides probe sets. As used herein, the term **"*probe set*"** refers to two or more probes which together allow detection of at least one CYP2C9 polymorphisms of interest (*e.g*., a SNP located within Amplicon p2 or Amplicon p3). In certain embodiments, the term "primer set" refers to a pair of allele-specific oligonucleotides (one wild-type (WT) and one mutant (MT) probes) that can be used in an ASPE reaction to detect a SNP of interest.

Examples of probe sets/pairs include Probe Set 1, which comprises a wild-type probe comprising SEQ ID NO. 5, or any active fragment thereof, and a mutant probe comprising SEQ ID NO. 6, or any fragment thereof. Probe Set 2(*3), which comprises a wild-type probe comprising SEQ ID NO. 7, or any active fragment thereof, and a mutant probe comprising SEQ ID NO. 8, or any fragment thereof. Probe Set 2(*4), which comprises a wild-type probe comprising SEQ ID NO. 9, or any active fragment thereof, and a mutant probe comprising SEQ ID NO. 10, or any fragment thereof. Probe Set 2(*5), which comprises a wild-type probe comprising SEQ ID NO. 11, or any active fragment thereof, and a mutant probe comprising SEQ ID NO. 12, or any fragment thereof.

The present invention further provides primer/probe sets. As used herein, the term **"*primer*/*probe set*"** refers to a combination comprising two or more primers which together are capable of priming the amplification of a CYP2C9 nucleotide sequence of interest to generate an amplification product (*e.g*., Amplicon p2 or Amplicon p3), and two or more probes which together allow detection of at least one CYP2C9 SNP located in the amplification product (*e.g*., a SNP within Amplicon p3). In certain embodiments, the term "primer/probe set" refers to a pair of forward primer and reverse primer that generate an amplification product of interest by PCR and at least one pair of allele-specific oligonucleotides (one wild-type probe and one mutant probe) that can be used in an ASPE reaction to detect a SNP that resides within the amplification product obtained by PCR. Several primer/probe sets may be used (for example assembled in a kit) for multiplex detection of CYP2C9 SNPs.

### Tag Sequences and Zipcode Sequences

The present invention further provides tag sequences and corresponding complementary zipcode sequences that can be used in detection methods (*e.g*., detection methods of the present invention). Tag sequences (and corresponding zipcode sequences) are sequences of nucleotides that do not significantly hybridize to a target nucleic acid molecule under assay conditions and may be used to test many different samples (*i.e.*, tag sequences are independent of the sequences being analyzed).

Exemplary tag sequences (R) and zipcode sequences (NC) are presented in Table 3. In certain embodiments, each probe is tagged with a unique inventive 25-mer sequence that is the reverse complement of a zipcode sequence that can be immobilized on a solid support. The orthogonal zipcode sequences were developed to capture the tag sequences of the wild-type and mutant of all four mutations with minimum cross interaction. The inventive zipcode sequences were designed to have similar Tms.

### Oligonucleotide Preparation

Oligonucleotides of the invention may be prepared by any of a variety of methods (see, for example, J. Sambrook et al., "Molecular Cloning: A Laboratory Manual", 1989, 2nd Ed., Cold Spring Harbour Laboratory Press: New York, NY; *"*PCR Protocols: A Guide to Methods and Applications", 1990, M.A. Innis (Ed.), Academic Press: New York, NY; P. Tijssen "Hybridization with Nucleic Acid Probes - Laboratory Techniques in Biochemistry and Molecular Biology (Parts I and II)", 1993, Elsevier Science; *"*PCR Strategies", 1995, M.A. Innis (Ed.), Academic Press: New York, NY; and *"*Short Protocols in Molecular Biology", 2002, F.M. Ausubel (Ed.), 5th Ed., John Wiley & Sons: Secaucus, NJ). For example, oligonucleotides may be prepared using any of a variety of chemical techniques well-known in the art, including, for example, chemical synthesis and polymerization based on a template as described, for example, in S.A. Narang et al., Meth. Enzymol. 1979, 68: 90-98; E.L. Brown et al., Meth. Enzymol. 1979, 68: 109-151; E.S. Belousov et al., Nucleic Acids Res. 1997, 25: 3440-3444; D. Guschin et al., Anal. Biochem. 1997, 250: 203-211; M.J. Blommers et al., Biochemistry, 1994, 33: 7886-7896; and K. Frenkel et al., Free Radic. Biol. Med. 1995, 19: 373-380; and U.S. Pat. No. 4,458,066.

For example, oligonucleotides may be prepared using an automated, solid-phase procedure based on the phosphoramidite approach. In such a method, each nucleotide is individually added to the 5'-end of the growing oligonucleotide chain, which is attached at the 3'-end to a solid support. The added nucleotides are in the form of trivalent 3'-phosphoramidites that are protected from polymerization by a dimethoxytriyl (or DMT) group at the 5'-position. After base-induced phosphoramidite coupling, mild oxidation to give a pentavalent phosphotriester intermediate and DMT removal provides a new site for oligonucleotide elongation. The oligonucleotides are then cleaved off the solid support, and the phosphodiester and exocyclic amino groups are deprotected with ammonium hydroxide. These syntheses may be performed on oligo synthesizers such as those commercially available from Perkin Elmer/Applied Biosystems, Inc. (Foster City, CA), DuPont (Wilmington, DE) or Milligen (Bedford, MA). Alternatively, oligonucleotides can be custom made and ordered from a variety of commercial sources well-known in the art, including, for example, the Midland Certified Reagent Company (Midland, TX), ExpressGen, Inc. (Chicago, IL), Operon Technologies, Inc. (Huntsville, AL), and many others.

Purification of the oligonucleotides of the invention, where necessary or desirable, may be carried out by any of a variety of methods well-known in the art. Purification of oligonucleotides is typically performed either by native acrylamide gel electrophoresis, by anion-exchange HPLC as described, for example, by J.D. Pearson and F.E. Regnier (J. Chrom., 1983, 255: 137-149) or by reverse phase HPLC (G.D. McFarland and P.N. Borer, Nucleic Acids Res., 1979, 7: 1067-1080).

The sequence of oligonucleotides can be verified using any suitable sequencing method including, but not limited to, chemical degradation (A.M. Maxam and W. Gilbert, Methods of Enzymology, 1980, 65: 499-560), matrix-assisted laser desorption ionization time-of-flight (MALDI-TOF) mass spectrometry (U. Pieles et al., Nucleic Acids Res., 1993, 21: 3191-3196), mass spectrometry following a combination of alkaline phosphatase and exonuclease digestions (H. Wu and H. Aboleneen, Anal. Biochem., 2001, 290: 347-352), and the like.

As already mentioned above, modified oligonucleotides may be prepared using any of several means known in the art. Non-limiting examples of such modifications include methylation, "caps", substitution of one or more of the naturally occurring nucleotides with an analog, and internucleotide modifications such as, for example, those with uncharged linkages (*e.g*., methyl phosphonates, phosphotriesters, phosphoroamidates, carbamates, etc), or charged linkages (*e.g*., phosphorothioates, phosphorodithioates, etc). Oligonucleotides may contain one or more additional covalently linked moieties, such as, for example, proteins (*e.g*., nucleases, toxins, antibodies, signal peptides, poly-L-lysine, etc), intercalators (*e.g*., acridine, psoralen, etc), chelators (*e.g.*, metals, radioactive metals, iron, oxidative metals, etc), and alkylators. The oligonucleotide may also be derivatized by formation of a methyl or ethyl phosphotriester or an alkyl phosphoramidate linkage. Furthermore, the oligonucleotide sequences of the present invention may also be modified with a label.

### Labeling of Oligonucleotide Sequences

In certain embodiments, the detection probes or amplification primers or both probes and primers are labeled with a detectable agent or moiety before being used in amplification/detection assays. In certain embodiments, the detection probes are labeled with a detectable agent. For example, a wild-type probe and mutant probe to be used for the ASPE-based detection of a SNP of interest may be labeled with two different detectable agents to allow for identification of the SNP. Preferably, a detectable agent is selected such that it generates a signal which can be measured and whose intensity is related (*e.g*., proportional) to the amount of amplification products in the sample being analyzed.

The association between the oligonucleotide and detectable agent can be covalent or non-covalent. Labeled detection probes can be prepared by incorporation of or conjugation to a detectable moiety. Labels can be attached directly to the nucleic acid sequence or indirectly (*e.g*., through a linker). Linkers or spacer arms of various lengths are known in the art and are commercially available, and can be selected to reduce steric hindrance, or to confer other useful or desired properties to the resulting labeled molecules (see, for example, E.S. Mansfield et al., Mol. Cell Probes, 1995, 9: 145-156).

Methods for labeling nucleic acid molecules are well-known in the art. For a review of labeling protocols, label detection techniques, and recent developments in the field, see, for example, L.J. Kricka, Ann. Clin. Biochem. 2002, 39: 114-129; R.P. van Gijlswijk et al., Expert Rev. Mol. Diagn. 2001, 1: 81-91; and S. Joos et al., J. Biotechnol. 1994, 35: 135-153. Standard nucleic acid labeling methods include: incorporation of radioactive agents, direct attachments of fluorescent dyes (L.M. Smith et al., Nucl. Acids Res., 1985, 13: 2399-2412) or of enzymes (B.A. Connoly and O. Rider, Nucl. Acids. Res., 1985, 13: 4485-4502); chemical modifications of nucleic acid molecules making them detectable immunochemically or by other affinity reactions (T.R. Broker et al., Nucl. Acids Res. 1978, 5: 363-384; E.A. Bayer et al., Methods of Biochem. Analysis, 1980, 26: 1-45; R. Langer et al., Proc. Natl. Acad. Sci. USA, 1981, 78: 6633-6637; R.W. Richardson et al., Nucl. Acids Res. 1983, 11: 6167-6184; D.J. Brigati et al., Virol. 1983, 126: 32-50; P. Tchen et al., Proc. Natl. Acad. Sci. USA, 1984, 81: 3466-3470; J.E. Landegent et al., Exp. Cell Res. 1984, 15: 61-72; and A.H. Hopman et al., Exp. Cell Res. 1987, 169: 357-368); and enzyme-mediated labeling methods, such as random priming, nick translation, PCR and tailing with terminal transferase (for a review on enzymatic labeling, see, for example, J. Temsamani and S. Agrawal, Mol. Biotechnol. 1996, 5: 223-232). More recently developed nucleic acid labeling systems include, but are not limited to: ULS (Universal Linkage System), which is based on the reaction of mono-reactive cisplatin derivatives with the N7 position of guanine moieties in DNA (R.J. Heetebrij et al., Cytogenet. Cell. Genet. 1999, 87: 47-52), psoralen-biotin, which intercalates into nucleic acids and upon UV irradiation becomes covalently bonded to the nucleotide bases (C. Levenson et al., Methods Enzymol. 1990, 184: 577-583; and C. Pfannschmidt et al., Nucleic Acids Res. 1996, 24: 1702-1709), photoreactive azido derivatives (C. Neves et al., Bioconjugate Chem. 2000, 11: 51-55), and DNA alkylating agents (M.G. Sebestyen et al., Nat. Biotechnol. 1998, 16: 568-576).

Any of a wide variety of detectable agents can be used in the practice of the present invention. Suitable detectable agents include, but are not limited to, various ligands, radionuclides (such as, for example, ³²P, ³⁵S, ³H, ¹⁴C, ¹²⁵I, ¹³¹I, and the like); fluorescent dyes (for specific exemplary fluorescent dyes, see below); chemiluminescent agents (such as, for example, acridinium esters, stabilized dioxetanes, and the like); spectrally resolvable inorganic fluorescent semiconductor nanocrystals (*i.e*., quantum dots), metal nanoparticles (*e.g*., gold, silver, copper and platinum) or nanoclusters; enzymes (such as, for example, those used in an ELISA, *i.e*., horseradish peroxidase, beta-galactosidase, luciferase, alkaline phosphatase); colorimetric labels (such as, for example, dyes, colloidal gold, and the like); magnetic labels (such as, for example, Dynabeads™); and biotin, dioxigenin or other haptens and proteins for which antisera or monoclonal antibodies are available.

In certain embodiments, the inventive detection probes are fluorescently labeled. Numerous known fluorescent labeling moieties of a wide variety of chemical structures and physical characteristics are suitable for use in the practice of this invention. Suitable fluorescent dyes include, but are not limited to, fluorescein and fluorescein dyes (*e.g*., fluorescein isothiocyanine or FITC, naphthofluorescein, 4',5'-dichloro-2',7'-dimethoxy-fluorescein, 6-carboxyfluorescein or FAM), carbocyanine, merocyanine, styryl dyes, oxonol dyes, phycoerythrin, erythrosin, eosin, rhodamine dyes (*e.g*., carboxytetramethylrhodamine or TAMRA, carboxyrhodamine 6G, carboxy-X-rhodamine (ROX), lissamine rhodamine B, rhodamine 6G, rhodamine Green, rhodamine Red, tetramethylrhodamine or TMR), coumarin and coumarin dyes (*e.g*., methoxycoumarin, dialkylaminocoumarin, hydroxycoumarin and aminomethylcoumarin or AMCA), Oregon Green Dyes (*e.g*., Oregon Green 488, Oregon Green 500, Oregon Green 514), Texas Red, Texas Red-X, Spectrum Red™, Spectrum Green™, cyanine dyes (*e.g.,* Cy-3™, Cy-5™, Cy-3.5™, Cy-5.5™), Alexa Fluor dyes (*e.g*., Alexa Fluor 350, Alexa Fluor 488, Alexa Fluor 532, Alexa Fluor 546, Alexa Fluor 568, Alexa Fluor 594, Alexa Fluor 633, Alexa Fluor 660 and Alexa Fluor 680), BODIPY dyes (*e.g*., BODIPY FL, BODIPY R6G, BODIPY TMR, BODIPY TR, BODIPY 530/550, BODIPY 558/568, BODIPY 564/570, BODIPY 576/589, BODIPY 581/591, BODIPY 630/650, BODIPY 650/665), IRDyes (*e.g*., IRD40, IRD 700, IRD 800), and the like. For more examples of suitable fluorescent dyes and methods for linking or incorporating fluorescent dyes to nucleic acid molecules see, for example, "The Handbook of Fluorescent Probes and Research Products", 9th Ed., Molecular Probes, Inc., Eugene, OR. Fluorescent dyes as well as labeling kits are commercially available from, for example, Amersham Biosciences, Inc. (Piscataway, NJ), Molecular Probes Inc. (Eugene, OR), and New England Biolabs Inc. (Berverly, MA).

A "tail" of normal or modified nucleotides can also be added to oligonucleotide probes for detectability purposes. A second hybridization with nucleic acid complementary to the tail and containing one or more detectable labels (such as, for example, fluorophores, enzymes or bases that have been radioactivity labeled or microparticles) allows visualization of the amplicon/probe hybrids (see, for example, the system commercially available from Enzo Biochem. Inc., New York: NY). Another example of an assay with which the inventive oligonucleotides are useful is a signal amplification method such as that described in U.S. Pat. No. 5,124,246 (which is incorporated herein by reference in its entirety). In that method, the signal is amplified through the use of amplification multimers, polynucleotides which are constructed so as to contain a first segment that hybridizes specifically to the "tail" added to the oligonucleotide probes, and a multiplicity of identical second segments that hybridize specifically to a labeled probe. The degree of amplification is theoretically proportional to the number of iterations of the second segment. The multimers may be either linear or branched. Branched multimers may be in the shape of a fork or a comb.

In certain embodiments of the present invention, the ASPE probes are each tagged with an inventive 25-mer tag sequence that is complementary to a zipcode sequence, which can be attached to a solid support. Examples of ASPE probes tagged with inventive 25-mer sequences are presented in Table 4.

The selection of a particular nucleic acid labeling technique will depend on the situation and will be governed by several factors, such as the ease and cost of the labeling method, the quality of sample labeling desired, the effects of the detectable moiety on the hybridization reaction (*e.g*., on the rate and/or efficiency of the hybridization process), the nature of the amplification method used, the nature of the detection system, the nature and intensity of the signal generated by the detectable label, and the like.

### II - Detection of CYP2C9 Polymorphisms

As already mentioned above, the oligonucleotide sequences of the present invention can be used in nucleic acid amplification methods for detecting the presence/absence of and/or for identifying CYP2C9 polymorphisms in a test sample obtained from an individual.

Detection methods of the present invention will generally include: preparation of a test sample comprising the CYP2C9 gene or genetic material comprising the CYP2C9 genomic DNA; amplification of at least one CYP2C9 target sequence using amplification primers provided herein (*e.g*., by PCR using a forward primer and a reverse primer) to produce an amplification product (*e.g*., Amplicon p2 and/or Amplicon p3); and detection of at least one CYP2C9 SNP that resides within the amplification product using detection probes provided herein (*e.g*., by ASPE using wild-type and mutant probes).

### Sample Preparation

Test samples suitable for use in detection methods of the present invention contain genetic material, *i.e*., DNA. Such DNA may be obtained from any cell source. Non-limiting examples of cell sources in clinical practice include, blood cells, buccal cells, cervico-vaginal cells, epithelial cells from urine, fetal cells, or any cells present in tissue obtained by biopsy. Cells may be obtained from body fluids (*e.g*., blood, serum, urine, sputum, saliva, cerebrospinal fluid, seminal fluid, lymph fluid, and the like), or tissues (*e.g*., skin, hair, buccal or conjunctival mucosa, muscles, bone marrow, lymph nodes, and the like). DNA from fetal or embryonic cells or tissues can be obtained by appropriate methods, such as amniocentesis or chorionic villus sampling.

Isolation, extraction or derivation of DNA may be carried out by any suitable method. Isolating DNA from a biological sample generally includes treating a biological sample in such a manner that genomic DNA present in the sample is extracted and made available for analysis. Any isolation method that results in extracted genomic DNA may be used in the practice of the present invention. It will be understood that the particular method used to extract DNA will depend on the nature of the source.

Methods of DNA extraction are well-known in the art. A classical DNA isolation protocol is based on extraction using organic solvents such as a mixture of phenol and chloroform, followed by precipitation with ethanol (J. Sambrook et al., "Molecular Cloning: A Laboratory Manual", 1989, 2nd Ed., Cold Spring Harbour Laboratory Press: New York, NY). Other methods include: salting out DNA extraction (P. Sunnucks et al., Genetics, 1996, 144: 747-756; S.M. Aljanabi and I. Martinez, Nucl. Acids Res. 1997, 25: 4692-4693), trimethylammonium bromide salts DNA extraction (S. Gustincich et al., BioTechniques, 1991, 11: 298-302) and guanidinium thiocyanate DNA extraction (J.B.W. Hammond et al., Biochemistry, 1996, 240: 298-300).

There are also numerous versatile kits that can be used to extract DNA from tissues and bodily fluids and that are commercially available from, for example, BD Biosciences Clontech (Palo Alto, CA), Epicentre Technologies (Madison, WI), Gentra Systems, Inc. (Minneapolis, MN), MicroProbe Corp. (Bothell, WA), Organon Teknika (Durham, NC), and Qiagen Inc. (Valencia, CA). User Guides that describe in great detail the protocol to be followed are usually included in all these kits. Sensitivity, processing time and cost may be different from one kit to another. One of ordinary skill in the art can easily select the kit(s) most appropriate for a particular situation.

In certain embodiments, methods of the present invention may be practiced on cellular material other than DNA. For example, polymorphisms that lie in the CYP2C9 gene may be detected in RNA.

Methods of RNA extraction are well known in the art (see, for example, J. Sambrook et al., "Molecular Cloning: A Laboratory Manual", 1989, 2nd Ed., Cold Spring Harbour Laboratory Press: New York) and several kits for RNA extraction from bodily fluids are commercially available, for example, from Ambion, Inc. (Austin, TX); Amersham Biosciences (Piscataway, NJ), BD Biosciences Clontech (Palo Alto, CA), BioRad Laboratories (Hercules, California), Dynal Biotech Inc. (Lake Success, NY), Epicentre Technologies (Madison, WI), Gentra Systems, Inc. (Minneapolis, MN), GIBCO BRL (Gaithersburg, MD), Invitrogen Life Technologies (Carlsbad, CA), MicroProbe Corp. (Bothell, WA), Organon Teknika (Durham, NC), Promega, Inc. (Madison, WI) and Qiagen Inc. (Valencia, CA).

Instead of being performed on extracted genetic material, detection methods of the present invention may be performed *in situ* directly upon tissue sections (fixed and/or frozen) of patient tissue obtained from biopsies or resection, such that no nucleic acid extraction/purification is necessary. Nucleic acid reagents may be used as probes and/or primers for such *in situ* procedures (see, for example, G.J. Nuova, "PCR in situ Hybridization: Protocols and Application", 1992, Raven Press: NY).

### Amplification of CYP2C9 Target Sequences Using Inventive Primers

The use of oligonucleotide sequences of the present invention to amplify CYP2C9 target sequences in test samples is not limited to any particular nucleic acid amplification technique or any particular modification thereof. In fact, the inventive oligonucleotide sequences can be employed in any of a variety of nucleic acid amplification methods well-known in the art (see, for example, A.R. Kimmel and S.L. Berger, Methods Enzymol. 1987, 152: 307-316; J. Sambrook et al., "Molecular Cloning: A Laboratory Manual", 1989, 2nd Ed., Cold Spring Harbour Laboratory Press: New York, NY; *"*Short Protocols in Molecular Biology", F.M. Ausubel (Ed.), 2002, 5th Ed., John Wiley & Sons: Secaucus, NJ).

Such nucleic acid amplification methods include, but are not limited to, the Polymerase Chain Reaction (or PCR, described, for example, in *"*PCR Protocols: A Guide to Methods and Applications", M.A. Innis (Ed.), 1990, Academic Press: New York; *"*PCR Strategies", M.A. Innis (Ed.), 1995, Academic Press: New York; *"Polymerase chain reaction: basic principles and automation in* PCR: A Practical Approach", McPherson et al. (Eds.), 1991, IRL Press: Oxford; Saiki et al., Nature, 1986, 324: 163; and U.S. Pat. Nos. 4,683,195, 4,683,202 and 4,889,818, each of which is incorporated herein by reference in its entirety); and reverse transcriptase polymerase chain reaction (or RT-PCR, described in, for example, U.S. Pat. Nos. 5,322,770 and 5,310,652).

The PCR (or polymerase chain reaction) technique is well-known in the art and has been disclosed, for example, in K.B. Mullis and F.A. Faloona, Methods Enzymol., 1987, 155: 350-355 and U.S. Pat. Nos. 4,683,202; 4,683,195; and 4,800,159 (each of which is incorporated herein by reference in its entirety). In its simplest form, PCR is an *in vitro* method for the enzymatic synthesis of specific DNA sequences, using two oligonucleotide primers that hybridize to opposite strands and flank the region of interest in the target DNA. A plurality of reaction cycles, each cycle comprising: a denaturation step, an annealing step, and a polymerization step, results in the exponential accumulation of a specific DNA fragment (*"*PCR Protocols: A Guide to Methods and Applications", M.A. Innis (Ed.), 1990, Academic Press: New York; *"*PCR Strategies", M.A. Innis (Ed.), 1995, Academic Press: New York; "Polymerase chain reaction: basic principles and automation in PCR: A Practical Approach", McPherson et al. (Eds.), 1991, IRL Press: Oxford; R.K. Saiki et al., Nature, 1986, 324: 163-166). The termini of the amplified fragments are defined as the 5' ends of the primers. Examples of DNA polymerases capable of producing amplification products in PCR reactions include, but are not limited to: *E. coli* DNA polymerase I, Klenow fragment of DNA polymerase I, T4 DNA polymerase, thermostable DNA polymerases isolated from *Thermus aquaticus* (Taq), available from a variety of sources (for example, Perkin Elmer), *Thermus thermophilus* (United States Biochemicals), *Bacillus stereothermophilus* (Bio-Rad), or *Thermococcus litoralis* ("Vent" polymerase, New England Biolabs). RNA target sequences may be amplified by reverse transcribing the mRNA into cDNA, and then performing PCR (RT-PCR), as described above. Alternatively, a single enzyme may be used for both steps as described in U.S. Pat. No. 5,322,770.

The duration and temperature of each step of a PCR cycle, as well as the number of cycles, are generally adjusted according to the stringency requirements in effect. Annealing temperature and timing are determined both by the efficiency with which a primer is expected to anneal to a template and the degree of mismatch that is to be tolerated. The ability to optimize the reaction cycle conditions is well within the knowledge of one of ordinary skill in the art. Although the number of reaction cycles may vary depending on the detection analysis being performed, it usually is at least 15, more usually at least 20, and may be as high as 60 or higher. However, in many situations, the number of reaction cycles typically ranges from about 20 to about 40.

The denaturation step of a PCR cycle generally comprises heating the reaction mixture to an elevated temperature and maintaining the mixture at the elevated temperature for a period of time sufficient for any double-stranded or hybridized nucleic acid present in the reaction mixture to dissociate. For denaturation, the temperature of the reaction mixture is usually raised to, and maintained at, a temperature ranging from about 85°C to about 100°C, usually from about 90°C to about 98°C, and more usually from about 93°C to about 96°C for a period of time ranging from about 3 to about 120 seconds, usually from about 5 to about 30 seconds.

Following denaturation, the reaction mixture is subjected to conditions sufficient for primer annealing to template DNA present in the mixture. The temperature to which the reaction mixture is lowered to achieve these conditions is usually chosen to provide optimal efficiency and specificity, and generally ranges from about 50°C to about 75°C, usually from about 55°C to about 70°C, and more usually from about 60°C to about 68°C. Annealing conditions are generally maintained for a period of time ranging from about 15 seconds to about 30 minutes, usually from about 30 seconds to about 5 minutes.

Following annealing of primer to template DNA or during annealing of primer to template DNA, the reaction mixture is subjected to conditions sufficient to provide for polymerization of nucleotides to the primer's end in a such manner that the primer is extended in a 5' to 3' direction using the DNA to which it is hybridized as a template, (*i.e*., conditions sufficient for enzymatic production of primer extension product). To achieve primer extension conditions, the temperature of the reaction mixture is typically raised to a temperature ranging from about 65°C to about 75°C, usually from about 67°C to about 73°C, and maintained at that temperature for a period of time ranging from about 15 seconds to about 20 minutes, usually from about 30 seconds to about 5 minutes.

The above cycles of denaturation, annealing, and polymerization may be performed using an automated device typically known as a thermal cycler or thermocycler. Thermal cyclers that may be employed are described in U.S. Pat. Nos. 5,612,473; 5,602,756; 5,538,871; and 5,475,610 (each of which is incorporated herein by reference in its entirety). Thermal cyclers are commercially available, for example, from Perkin Elmer-Applied Biosystems (Norwalk, CT), BioRad (Hercules, CA), Roche Applied Science (Indianapolis, IN), and Stratagene (La Jolla, CA).

In addition to the enzymatic thermal amplification technique described above, well-known isothermal enzymatic amplification reactions can be employed to amplify CYP2C9 target sequences using oligonucleotide primers of the present invention (S.C. Andras et al., Mol. Biotechnol., 2001, 19: 29-44). These methods include, but are not limited to, Transcription-Mediated Amplification (or TMA, described in, for example, D.Y. Kwoh et al., Proc. Natl. Acad. Sci. USA, 1989, 86: 1173-1177; C. Giachetti et al., J. Clin, Microbiol., 2002, 40: 2408-2419; and U.S. Pat. No. 5,399,491); Self-Sustained Sequence Replication (or 3SR, described in, for example, J.C. Guatelli et al., Proc. Natl. Acad. Sci. USA, 1990, 87: 1874-1848; and E. Fahy et al., PCR Methods and Applications, 1991, 1: 25-33); Nucleic Acid Sequence Based Amplification (or NASBA, described in, for example, T. Kievits et al., J. Virol., Methods, 1991, 35: 273-286; and U.S. Pat. No. 5,130,238) and Strand Displacement Amplification (or SDA, described in, for example, G.T. Walker et al., PNAS, 1992, 89: 392-396; EP 0 500 224 A2). Each of the references cited in this paragraph is incorporated herein by reference in its entirety.

Amplification products obtained using primers of the present invention may be detected using agarose gel electrophoresis and visualization by ethidium bromide staining and exposure to ultraviolet (UV) light or by sequence analysis of the amplification product.

### Allele Specific Primer Extension Reaction

SNPs located within Amplicon p2 and Amplicon p3 can be specifically detected by allele-specific primer extension (ASPE) using detection probes comprising SEQ ID NOs. 5 through 12.

In ASPE, the presence or absence of a particular SNP is detected by selective allele-specific probe extension, wherein one of the allele-specific probes is extended without extension of the other allele-specific probe(s). In these methods, allele-specific probes are used to anneal to the target; the 3'-terminal base of the allele-specific probes is complementary to the corresponding template base of one allele but is a mismatch for the alternative allele(s). Since the extension starts at the 3'-end of the probe, a mismatch at or near this position has an inhibitory effect on extension; and DNA polymerases extend probes with a mismatched 3' nucleotide at a much lower efficiency than probes with perfect matches. Therefore, under appropriate conditions, only that allele-specific probe which is complementary to the target is extended (e.g., with biotinylated nucleotides).

Methods of using allele-specific oligonucleotides, such as those described herein, have been extensively described (see, for example, C.R. Newton et al., Nucl. Acids Res., 1989, 17: 2503-2516; W.C. Nichols et al., Genomics, 1989, 5: 535-540; D.Y. Wu, Proc. Natl. Acad. Sci. USA, 1989, 86: 2757-2760; C Dutton and S.S. Sommer, Biotechniques, 1991, 11: 700-702; R.S. Cha et al., PCR Methods Appl., 1992, 2: 14-20; L. Ugozzoli and R.B. Wallace, Methods Enzymol., 1991, 2: 42-48).

As will be recognized by one skilled in the art, in certain methods of the present invention, a single primer or a set of primers (e.g., forward and reverse primers) can be used depending on whether primer extension, linear or exponential amplification of the template is desired. When a single primer is used, the primer is typically an allele-specific probe, as described herein. When two primers are used, one is an allele-specific primer and the other is a complementary strand primer which anneals to the other DNA strand distant from the allele-specific primer. A set of primer pairs, wherein each pair comprises an allele-specific primer and a complementary strand primer, can also be used to distinguish alleles of a particular SNP. For example, the allele-specific primers of a set can be unique with respect to each other: one of the allele-specific primers may be complementary to the wild-type allele (*i.e*., allele-specific to the normal allele), and the others may be complementary to the alternative alleles. Each of the allele-specific primers in such a set may be paired with a common complementary strand primer. Multiple sets of pairs of primers can be used for the multiplex detection of SNPs.

In an ASPE reaction according to the present invention, amplification products (e.g., Amplicon p2 and/or Amplicon p3) are generally combined with allele-specific primers (e.g., one wild-type oligonucleotide sequence and one mutant oligonucleotide sequence provided herein), deoxyribonucleoside triphosphates (dNTPs), biotinylated dNTPs, a thermostable nucleic acid polymerase that lacks 3' nuclease activity, and an aqueous buffer medium to form a primer extension reaction mixture.

The term ***"thermostable",*** when used herein in reference to a nucleic acid polymerase, refers to an enzyme which is stable and active at a temperature as great as between about 90°C and about 100°C, or between about 70°C and about 98°C. A representative thermostable nucleic acid polymerase isolated from *Thermus aquaticus* (Taq) is described in U.S. Pat. No. 4,889,818 and a method for using it in conventional PCR is described in R.K. Saiki et al., Science, 1988, 239: 487-491. Another representative thermostable nucleic acid polymerase, isolated from P. *furiosus* (Pfu), is described in K.S. Lundberg et al., Gene, 1991, 108: 1-6. Additional examples of thermostable polymerases include polymerases extracted from the thermophilic bacteria *Thermus flavus, Thermus ruber*, *Thermus thermophilus*, *Bacillus stearothermophilus, Thermus lacteus, Thermus rubens, Thermotoga maritima,* or from thermophilic archaea *Thermococcus litoralis* and *Methanothermus fervidus.* Thermostable DNA polymerases suitable for use in the practice of the present invention include, but are not limited to, *E*. *coli* DNA polymerase I, *Thermus thermophilus* (Tth) DNA polymerase, *Bacillus stearothermophilus* DNA polymerase, *Thermococcus litoralis* DNA polymerase, *Thermus aquaticus* (Taq) DNA polymerase and *Pyrococcus furiosus* (Pfu) DNA polymerase.

In certain embodiments, the primer extension reaction mixture comprises a thermostable nucleic acid polymerase lacking 3'→5' exonuclease activity or lacking both 5'→3' and 3'→5' exonuclease activity. Lacking 3'→5' exonuclease activity is crucial to the specificity of the ASPE reaction because the 3' end of ASPE probes should no be subjected to any modification by polymerase. With such nucleic acid polymerases, the target DNA is used as a template for extending the allele-specific oligonucleotide and no extension occurs if there is a mismatch at the terminal 3' end of the allele-specific oligonucleotide.

Examples of nucleic acid polymerases substantially lacking 5'→3' exonuclease activity include, but are not limited to, Klenow and Klenow exo-, and T7 DNA polymerase (Sequenase). Examples of thermostable nucleic acid polymerases substantially lacking 5'→3' exonuclease activity include, but are not limited to, Pfu, the Stoffel fragment of Taq, N-truncated Bst, N-truncated Bca, Genta, JdF3 exo, Vent, Deep Vent, UlTma and ThermoSequenase. Examples of thermostable nucleic acid polymerases substantially lacking both 5'→3' and 3'→5' exonuclease activity include, but are not limited to, exo-Pfu (a mutant form of Pfu), Vent exo (a mutant form of Vent), and Deep Vent exo- (a mutant form of Deep Vent). Thermostable nucleic acid polymerases are commercially available for example from Stratagene (La Jolla, CA), New England BioLabs (Ipswich, MA), BioRad (Hercules, CA), Perkin-Elmer (Wellesley, MA), and Hoffman-LaRoche (Basel, Switzerland).

A primer extension reaction mixture generally comprises enough thermostable polymerase so that conditions suitable for enzymatic primer extension are maintained during all the reaction cycles. Alternatively, polymerase may be added to the primer extension reaction mixture after a certain number of reaction cycles have been performed.

The aqueous buffer medium used in an ASPE reaction generally acts as a source of monovalent ions, divalent cations, and buffer agent. Any convenient source of monovalent ions, such as potassium chloride, potassium acetate, potassium glutamate, ammonium acetate, ammonium chloride, ammonium sulfate, and the like may be employed. The divalent cation may be magnesium, manganese, zinc and the like. Magnesium (Mg²⁺) is often used. Any source of magnesium cations may be employed, including magnesium chloride, magnesium acetate, and the like. The amount of Mg²⁺ present in the buffer may range from about 0.5 to about 10 mM. Representative buffering agents, or salts that may be present in the buffer include Tris, Tricine, HEPES, MOPS, and the like. The amount of buffering agent generally ranges from about 5 to about 150 mM. In certain embodiments, the buffer agent is present in an amount sufficient to provide a pH ranging from about 6.0 to 9.5, most preferably about pH 7.3. Other agents which may be present in the buffer medium include chelating agents, such as EDTA, EGTA and the like.

Generally, the primer extension reaction mixture will comprise four different types of dNTPs corresponding to the four naturally occurring bases, i.e., dATP, dTTP, dCTP, and dGTP. In certain embodiments, the primer extension mixture additionally contains biotinylated dNTPs, for example biotinylated dCTP, for incorporation of biotin in the primer extension product(s). The resulting biotinylated primer extension products may subsequently be exposed to a streptavidin-dye complex for detection purposes, as is well-known in the art. Examples of streptavidin-dye complexes suitable for use in the practice of the methods of the present invention include, but are not limited to, steptavidin-fluorescein (SA-FITC), streptavidin-phycoerythrin (SA-PE), streptavidin-rhodamine B (SA-R), streptavidin-Texas Red (SA-TR), streptavidin-phycocyanin (SA-PC), and streptavidin-allophycocyanine (SA-APC).

In preparing a primer extension reaction mixture, the various constituent components may be combined in any convenient order.

Following addition of all the components, the ASPE reaction mixture is subjected to primer extension reaction conditions, *i.e.,* to conditions that allow for polymerase-mediated primer extension by addition of nucleotides to the end of the annealed (*i.e*., hybridized) primer molecule using the target strand as a template. In many embodiments, the primer extension reaction conditions are similar to PCR amplification conditions (see above).

In methods of the present invention, ASPE reactions may be performed under homogeneous or heterogeneous conditions. In a homogeneous ASPE reaction, all the reagents are in solution. Alternatively, detection probes capable of hybridizing specifically to allelic variants may be attached to a solid support. In some embodiments, such a solid support may be in the form of a chip or array. The solid support may be contacted with the PCR reaction mixture (*e.g*., containing Amplicon p2 and/or Amplicon p3), and amplification products in the PCR reaction mixture are allowed to hybridize to one or more probes attached to the solid support. Primer extension may be performed after hybridization, as described above, for example using one or more labeled nucleotides. In other embodiments, each detection probe is attached to a microbead. The bead-labeled detection probes may be added to the PCR reaction mixture, and amplification products in the PCR reaction mixture are allowed to hybridize to one or more probes. Primer extension may be performed after hybridization, as described above.

### Detection of SNPs in Primer Extension Products

Analysis of primer extension products can be accomplished using any of a wide variety of methods.

Following primer extension performed under homogeneous conditions, it may be desirable to separate the primer extension products from each other and from other components of the reaction mixture (*e.g*., unamplified DNA, excess primers/probes, etc) for purpose of analysis. In certain embodiments, separation of primer extension products is accomplished by employing capture reagents. Capture reagents typically consist of a solid support material coated with one or more binding members specific for the same or different binding partners. The term ***"solid support material",*** as used herein, refers to any material which is insoluble or can be made insoluble by a subsequent reaction or manipulation. Solid support materials can be latex, plastic, derivatized plastic, magnetic or non-magnetic metal, glass or silicon surface or surfaces of test tubes, microtiter wells, sheets, beads, microparticles, chips and other configurations known to those of ordinary skill in the art. To facilitate separation and/or detection of primer extension products, an extension primer can be labeled with a binding member (*e.g*., a tag sequence provided herein) that is specific for its binding partner which is attached to a solid material (*e.g*., its complementary zipcode sequence provided herein). The primer extension products can be separated from other components of the extension reaction mixture by contacting the mixture with a solid support, and then removing, from the reaction mixture, the solid support to which extension products are bound, for example, by filtration, sedimentation, washing or magnetic attraction.

For example, an allele-specific oligonucleotide can be coupled with a moiety that allows affinity capture, while other allele-specific oligonucleotides remain unmodified or are coupled with different affinity moieties. Modifications can include a sugar (for binding to a solid phase material coated with lectin), a hydrophobic group (for binding to a reverse phase column), biotin (for binding to a solid phase material coated with streptavidin), or an antigen (for binding to a solid phase material coated with an appropriate antibody). Extension reaction mixtures can be run through an affinity column, the flow-through fraction collected, and the bound fraction eluted, for example, by chemical cleavage, salt elution, and the like. Alternatively, extension reaction mixtures can be contacted with affinity capture beads.

Alternatively, each allele-specific oligonucleotide may comprise a nucleotide sequence (binding member) at its 5' terminus, that is complementary to a nucleotide sequence (binding partner) attached to a solid support. Allele-specific oligonucleotides used in detection methods of the present invention may be coupled to an identical tag sequence (*e.g.,* universal capture sequence) complementary to a tag probe sequence *(e.g.,* corresponding zipcode sequence) attached to a solid support. Alternatively, each allele-specific oligonucleotide may comprise a tag sequence that is allele-specific and complementary to a tag probe sequence attached to a solid support. The tag may be, for example, about 10 to about 30 nucleotides in length. Tags and specific sets of tags and tag probe sequences are disclosed, for example, in U.S. Pat. No. 6,458,530 (which is incorporated herein by reference in its entirety). In general, tag and tag sequences are selected such that they are not present in the genome (or part of the genome) of interest in order to prevent cross-hybridization with the genome. Tags are often selected in sets; and tags in a set are generally selected such that they do not cross-hybridize with another tag in the set or with the complement of another tag in the set. Tag probe sequence may be attached to multiple microparticles or to an array or micro-array. An array or micro-array may be prepared to contain a plurality of probe elements. For example, each probe elements may include a plurality of tag probes that comprise substantially the same sequence that may be of different lengths. Probe elements on an array may be arranged on the solid surface at different densities.

In certain embodiments of the present invention, each allele-specific extension probe is tagged with an inventive 25-mer tag sequence (R) which is complementary to a zipcode sequence (NC) attached to a solid support, wherein the tag and zipcode sequences are selected from those presented in Table 3.

Methods of attaching (or immobilizing) tag sequences to a solid support are known in the art (see, for example, J. Sambrook et al., "Molecular Cloning: A Laboratory Manual", 1989, 2nd Ed., Cold Spring Harbour Laboratory Press: New York, NY; *"*Short Protocols in Molecular Biology", 2002, F.M. Ausubel (Ed.), 5th Ed., John Wiley & Sons; U. Maskos and E.M. Southern, Nucleic Acids Res., 1992, 20: 1679-1684; R.S. Matson et al., Anal. Biochem., 1995, 224; 110-116; R.J. Lipshutz et al., Nat. Genet., 1999, 21: 20-24; Y.H. Rogers et al., Anal. Biochem., 1999, 266: 23-30; M.A. Podyminogin et al., Nucleic Acids Res., 2001, 29: 5090-5098; Y. Belosludtsev et al., Anal. Biochem., 2001, 292: 250-256; U.S. Pat. Nos. 5,427,779, 5,512,439, 5,589,586, 5,716,854 and 6,087,102). Alternatively, one can rely on commercially available systems including arrays and microarrays, such as those developed, for example, by Affymetrix, Inc. (Santa Clara, CA) and Illumina, Inc. (San Diego, CA); and multiplexed bead- and particle-based systems such as those developed by BD Biosciences (Bedford, MA) and Luminex, Corp. (Austin, TX).

After heterogeneous ASPE or after separation of extension products from other components of the ASPE reaction mixture (as described above), the presence or absence of extension products (indicative of the presence or absence of particular SNPs in the DNA sample under investigation) can be detected using any of a wide variety of methods, including spectroscopic, photochemical, biochemical, immunochemical, electrical, optical, radiochemical, and chemical methods. Selection of a method of detection will generally depend on several factors including, but not limited to, the type of assay carried out (*e.g*., single-plex *vs*. multiplex), the separation technique used, the presence or absence of a label (*i.e*., detectable moiety) on the extension products, and the nature of the labels (*e.g*., directly *vs*. indirectly detectable), if present.

Primer extension products generated by methods of the present invention may be detected through hybridization. For example, the extension products may be contacted with labeled nucleic acid probes. For example, each nucleic acid probe may be specific for an extension product (indicative of one allele of a SNP of interest) and may be labeled with a detectable moiety that is different from the detectable moieties carried by the other nucleic acid probes used in the assay, thereby allowing multiplex SNP detection.

Primer extension products bound to microparticles (also called microbeads) can be detected using different methods. For example, in multiplexed assays of the present invention, extension products can be simultaneously detected using pre-coded microbeads. Beads may be pre-coded using specific bead sizes, different colors and/or color intensities, different fluorescent dyes or fluorescent dye combinations.

Color-coded microspheres can be made using any of a variety of methods such as those disclosed in U.S. Pat. Nos. 6,649,414; 6,514,295; 5,073,498; 5,194,300; 5,356,713; 4,259,313; 4,283,382 and the references cited in these patents. Color-coded microspheres are also commercially available, for example, from Cortex Biochem., Inc. (San Leandro, CA); Seradyn, Inc. (Indianapolis, IN); Dynal Biotech, LLC (Brown Deer, WI); Spherotech, Inc. (Libertyville, IL); Bangs Laboratories, Inc. (Fishers, IN); and Polysciences, Inc. (Warrington, PA).

For example, polystyrene microspheres are provided by Luminex Corp. (Austin, TX) that are internally dyed with two spectrally distinct fluorescent dyes (x-MAP™ microbeads). Using precise ratios of these fluorophores, a large number of different fluorescent bead sets can be produced (e.g., 100 sets). Each set of beads can be distinguished by its code (or spectral signature), a combination of which allows for detection of a large number of different extension products in a single reaction vessel. The magnitude of the biomolecular interaction that occurs at the microsphere surface is measured using a third fluorochrome that acts as a reporter. These sets of fluorescent beads with distinguishable codes can be used to label extension products. Labeling (or attachment) of extension products to beads can be by any suitable means including, but not limited to, chemical or affinity capture, cross-linking, electrostatic attachment, and the like. In certain embodiments, labeling is carried out through hybridization of allele-specific tag and tag probe sequences, as described above. Because each of the different extension products is uniquely labeled with a fluorescent bead, the captured extension product (indicative of one allele of a SNP of interest) will be distinguishable from other different extension products (including extension products indicative of other alleles of the same SNP and extension products indicative of other SNPs of interest). Following tag/tag probe hybridization, the microbeads can be analyzed using different methods such as, for example, flow cytometry-based methods.

Extension products bound to microbeads can be detected using flow cytometry. Flow cytometry is a sensitive and quantitative technique that analyzes particles in a fluid medium based on the particles' optical characteristic (H.M. Shapiro, "Practical Flow Cytometry", 3rd Ed., 1995, Alan R. Liss, Inc.; and *"*Flow Cytometry and Sorting, Second Edition", Melamed et al. (Eds), 1990, Wiley-Liss: New York). A flow cytometer hydrodynamically focuses a fluid suspension of particles containing one or more fluorophores, into a thin stream so that the particles flow down the stream in a substantially single file and pass through an examination or analysis zone. A focused light beam, such as a laser beam, illustrates the particles as they flow through the examination zone, and optical detectors measure certain characteristics of the light as it interacts with the particles *(e.g.,* light scatter and particle fluorescence at one or more wavelengths). In the stream, the microbeads are interrogated individually as they pass the detector and high-speed digital signal processing classifies each bead based on its code and quantifies the reaction on the bead surface. Thousands of beads can be interrogated per second, resulting in a high-speed, high-throughput and accurate detection of multiple different SNPs. In embodiments where the extension reaction is carried out in the presence of biotinylated dNTPs, the reaction between beads and extension products may be quantified by fluorescence after reaction with fluorescently-labeled streptavidin *(e.g.,* Cy5-streptavidin conjugate). Instruments for performing such assay analyses are commercially available, for example, from Luminex (*e.g*., Luminex^{®} 100™ Total System, Luminex^{®} 100™ IS Total System, Luminex^{®}. High Throughput Screening System).

Extension products bound to arrays, micro-arrays or chips can be detected using different methods. In certain embodiments, primer extension products are captured (or attached) *via* hybridization to probes on array sites (as mentioned above). This attachment is generally a direct hybridization between an adapter sequence on the primer extension product *(e.g.,* an allele-specific tag sequence) and a corresponding capture probe *(e.g.,* complementary zipcode sequence) immobilized onto the surface of the array. Alternatively, the attachment can rely on indirect "sandwich" complexes using capture extender probes as known in the art (see, for example, M. Ranki et al., Gene, 1983, 21: 77-85; B.J. Connor et al., Proc. Natl. Acad. Sci. USA, 1983, 80: 278-282; and U.S. Pat. Nos. 4,563,419 and 4,751,177). The presence or absence of a bound extension product at a given spot (or position) on the array is generally determined by detecting a signal *(e.g.,* fluorescence) from the label coupled to the product. Furthermore, since the sequence of the capture probe at each position on the array is known, the identity of an extension product at that position can be determined.

Extension products bound to arrays are often (directly or indirectly) fluorescently labeled. Methods for the detection of fluorescent labels in array configurations are known in the art and include the use of "array reading" or "scanning" systems, such as charge-coupled devices (*i.e.*, CCDs). Any known device or method, or variation thereof can be used or adapted to practice the methods of the invention (see, for example, Y. Hiraoka et al., Science, 1987, 238: 36-41; R.S. Aikens et al., Meth. Cell Biol. 1989, 29: 291-313; A. Divane et al., Prenat. Diagn. 1994, 14: 1061-1069; S.M. Jalal et al., Mayo Clin. Proc. 1998, 73: 132-137; V.G. Cheung et al., Nature Genet. 1999, 21: 15-19; see also, for example, U.S. Pat. Nos. 5,539,517; 5,790,727; 5,846,708; 5,880,473; 5,922,617; 5,943,129; 6,049,380; 6,054,279; 6,055,325; 6,066,459; 6,140,044; 6,143,495; 6,191,425; 6,252,664; 6,261,776 and 6,294,331).

Commercially available microarray scanners are typically laser-based scanning systems that can acquire one (or more than one) fluorescent image (such as, for example, the instruments commercially available from PerkinElmer Life and Analytical Sciences, Inc. (Boston, MA), Virtek Vision, Inc. (Ontario, Canada) and Axon Instruments, Inc. (Union City, CA)). Arrays can be scanned using different laser intensities in order to ensure the detection of weak fluorescence signals and the linearity of the signal response at each spot on the array. Fluorochrome-specific optical filters may be used during acquisition of the fluorescent images. Filter sets are commercially available, for example, from Chroma Technology Corp. (Rockingham, VT).

A computer-assisted image analysis system is generally used to analyze fluorescent images acquired from arrays. Such systems allow for an accurate quantitation of the intensity differences and for an easy interpretation of the results. A software for fluorescence quantitation and fluorescence ratio determination at discrete spots on an array is usually included with the scanner hardware. Softwares and/or hardwares are commercially available and may be obtained from, for example, Affymetrix, Inc. (Santa Clara, CA), Applied Spectral Imaging, Inc. (Carlsbad, CA), Chroma Technology Corp. (Rockingham, VT), Leica Microsystems (Bannockburn, IL), and Vysis, Inc. (Downers Grove, IL).

Alternatively, a planar waveguide (PWG) chip technique can be used to detect surface bound fluorescently-labeled extension products. A waveguide refers to a two dimensional total internal reflection (TIR) element that provides an interface capable of internal reference at multiple points, thereby creating an evanescent wave that is substantially uniform across all or nearly all the entire surface. The waveguide can be comprised of transparent material such as glass, quartz, plastics such as polycarbonate, acrylic or polystyrene. The glass or other types of surfaces used for waveguides can be modified with any of a variety of functional groups including binding members such as haptens or oligonucleotide sequences (e.g., zipcode sequences).

In PWG, fluorescent excitation is carried out using an exponentially decaying evanescent light field, which preferentially excites labeled molecules that are captured within the field. Since molecules in solution (*i.e*., non surface bound) are not within the evanescent field, they do not get excited. This technique presents several advantages including very low fluorescent background, high dynamic range, and allows measurements in turbid solutions or optically dense suspensions. Multiplexed detection can be achieved by combining 2D arrays of ligands and CCD camera detection.

### Controls

In certain embodiments of the invention, an internal control or internal standard is added to the biological sample (or to purified/isolated nucleic acid extracted from the biological sample) to serve as a control for extraction and/or target amplification. Preferably, the internal control includes a sequence that differs from the target sequence(s), and is capable of amplification by the primers used to amplify the target sequence(s). The use of an internal control allows for the monitoring of the extraction process, amplification reaction, and detection, and control of the assay performance. The amplified control and amplified target(s) are typically distinguished at the detection step by using different probes (e.g., labeled with different detectable agents) for the detection of the control and target. As will be appreciated by one of ordinary skill in the art, more than one internal control can be used.

### Multiplex Detection of CYP2C9 Polymorphisms

In certain embodiments, the methods of the present disclosure are used to determine the genotype of an individual with respect to both CYP2C9 alleles present in that individual's genome. In some embodiments, the methods of the present invention are used to detect the presence of multiple polymorphic variants (*e.g*., polymorphic variants at a plurality of polymorphic sites) in parallel or otherwise substantially simultaneously.

Oligonucleotide arrays represent one suitable means for doing so. Methods can also be used in which detection probes are attached to microparticles or are modified to be capable of attachment to microparticles (as described above). Other methods, including methods in which reactions (*e.g*., amplification, detection) are performed in individual vessels (*e.g*., within individual wells of a multi-well plate or other vessel) may also be performed so as to detect the presence/identity of multiple polymorphic variants (*e.g*., polymorphic variants at a plurality of polymorphic sites) in parallel or substantially simultaneously.

Using such methods, the presence or absence of a plurality of polymorphic variants at different polymorphic sites can be detected. Thus, a genetic profile for an individual can be generated, wherein the genetic profile indicates which allelic variant is present at a plurality of different CYP2C9 polymorphisms that are associated with adverse drug reaction.

### III - Uses of Inventive Oligonucleotide Sequences and Detection Methods

The present disclosure provides a variety of methods for determining the identity of CYP2C9 allele(s) present in an individual. The inventive methods can be used, for example, to predict how such an individual will respond to drugs or other xenobiotic compounds that are metabolized, at least in part, by CYP2C9.

As but one limiting example, an individual that carries one or more "defective" CYP2C9 alleles, which defective alleles do not function to metabolize one or more particular drugs, may be susceptible to toxicity and/or to an otherwise adverse drug reaction since such an individual will be unable to metabolize the drugs as quickly as an individual carrying one or more normal CYP2C9 alleles, and the active, non-metabolized drug will remain in the individual's system for a longer period of time.

Thus, determining that an individual carries on or more such defective CYP2C9 alleles can be used to predict whether such an individual is susceptible to toxicity and/or to an otherwise adverse drug reaction. In certain embodiments, determining that an individual carries one or more such defective CYP2C9 alleles can be used to select an appropriate therapeutic regimen including, but not limited to, selecting one or more appropriate drugs, modulating drug dose, modulating dosing interval, etc. In certain embodiments, an individual that carries one or more such defective CYP2C9 alleles can be administered a different drug or other therapeutic regimen, such that any potential toxicity is avoided altogether.

In certain embodiments, a drug is administered to an individual in a pro-drug form in which the administered drug itself exhibits little or no activity. However, such a drug may be subject to metabolization by CYP2C9 such that upon metabolization, an active metabolic product is generated. In such embodiments, an individual carrying one or more "defective" CYP2C9 alleles may exhibit complete or partial immunity to a therapeutic regimen based on that drug since less metabolic product, or no metabolic product, will be generated.

In certain embodiments of the present invention, a panel of CYP2C9 polymorphisms (e.g., two or more SNPs) is defined that provides diagnostic and/or prognostic information when an individual is genotyped.with respect to the SNPs. In certain embodiments, results obtained from the panel predict the risk of developing adverse drug response. The risk can be, *e.g.,* absolute risk, which can be expressed in terms of likelihood *(e.g.,* % likelihood) that an individual will experience adverse drug response. The risk can be expressed in terms of relative risk, *e.g.,* a factor that expresses the degree to which the individual is at increased risk relative to the risk the individual would face if his or her genotype with respect to one or more of the polymorphisms was different. Individuals can be stratified based on their risk. Such stratification can be used, for example, to select individuals who would be likely to benefit from particular therapeutic regimens. It should be emphasized that the information provided by the methods of the present invention can be qualitative or quantitative and can be expressed using any convenient means.

It will be appreciated by one skilled in the art that the risk obtained using methods according to the present invention may be compared to and/or combined with results from other tests or assays performed for determining the susceptibility of an individual to toxicity and/or otherwise adverse drug reaction. Such comparison and/or combination may help to guide specific and individualized therapy, *e.g.,* to optimize treatment and avoid drug adverse response.

### IV - Kits

The present invention provides kits comprising materials useful for the detection and identification of CYP2C9 polymorphisms according to methods described herein. The inventive kits may be used by diagnostic laboratories, experimental laboratories, or practitioners. The invention provides kits which can be used in these different settings.

Materials and reagents useful for the detection of CYP2C9 polymorphisms according to the present invention may be assembled together in a kit. In certain embodiments, an inventive kit comprises at least one inventive primer set and/or primer/probe set, and optionally, amplification reaction reagents and/or primer extension reagents. Each kit necessarily comprises the reagents which render the procedure specific. Thus, a kit intended to be used for the detection of a particular SNP preferably comprises oligonucleotide sequences described herein that can be used to amplify a CYP2C9 target sequence that comprises the particular SNP and oligonucleotide sequences described herein that can be used in ASPE for detecting the SNP of interest. A kit intended to be used for the multiplex detection of a plurality of SNPs preferably comprises a plurality of oligonucleotide sequences described herein that can be used to amplify CYP2C9 target sequences that comprise the SNPs and oligonucleotide sequences described herein that can be used in ASPE reactions to detect the SNPs of interest.

Suitable Amplification/primer extension reaction reagents that can be included in an inventive kit include, for example, one or more of: buffers; enzymes having reverse transcriptase and/or polymerase activity or exonuclease activity; enzymes having polymerase activity and lacking 3'→5' exonuclease activity or both 5'→3' and 3'→5' exonuclease activity; enzyme cofactors such as magnesium or manganese; salts; nicotinamide adenide dinuclease (NAD); and deoxynucleoside triphosphates (dNTPs) such as, for example, deoxyadenosine triphospate; deoxyguanosine triphosphate, deoxycytidine triphosphate and thymidine triphosphate, biotinylated dNTPs, suitable for carrying out the amplification/ASPE reactions.

Depending on the procedure, an inventive kit may further comprise one or more of: wash buffers and/or reagents; hybridization buffers and/or reagents; labeling buffers and/or reagents; and detection means. Buffers and/or reagents are preferably optimized for the particular amplification/detection technique for which the kit is intended. Protocols for using these buffers and reagents for performing different steps of the procedure may also be included in the kit.

Furthermore, a kit may be provided with an internal control as a check on the amplification procedure to prevent occurrence of false negative test results due to failures in amplification. An optimal control sequence is selected in such a way that it will not compete with the target nucleic acid sequence(s) in the amplification reaction (as described above).

Kits may also contain reagents for the isolation of nucleic acids from biological samples prior to amplification and/or reagents for the separation/purification of amplified CYP2C9 target sequence(s) of interest.

Reagents may be supplied in a solid *(e.g.,* lyophilized) or liquid form. The kits of the present invention optionally comprise different containers *(e.g.,* vial, ampoule, test tube, flash, or bottle) for each individual buffer and/or reagent. Each component will generally be suitable as aliquoted in its respective container or provided in a concentrated form. Other containers suitable for conducting certain steps of the amplification/detection assay may also be provided. The individual containers of the kit are preferably maintained in close confinement for commercial use.

In embodiments where the kit comprises primers and/or probes suitable for detection of a plurality of CYP2C9 polymorphic variants, the probes (or zipcode sequences complementary to tag sequences present on the probes) may be covalently or non-covalently attached to microparticles *(e.g.,* beads). Alternatively, the probes (or zipcode sequences complementary to tag sequences present on the probes) may be covalently or non-covalently attached to a substantially planar, rigid substrate or support. The substrate may be transparent to radiation of the excitation and emission wavelengths used for excitation and detection of typical labels *(e.g.,* fluorescent labels, quantum dots, plasmon resonant particles, nanoclusters), *e.g.,* 400 to 900 nm. Materials such as glass, plastic, quartz, etc. are suitable. For example, a glass slide or the like can be used.

In certain embodiments, the kits of the invention are adaptable to high-throughput and/or automated operation. For example, the kits may be suitable for performing assays in multi-well plates and may utilize automated fluid handling and/or robotic systems, plate readers, etc. In some embodiments, flow cytometry is used.

One of ordinary skill in the art will appreciate that a number of other polymorphisms associated with adverse drug response are known in the art, including other CYP2C9 polymorphisms as well as polymorphisms of other cytochrome P540 genes. In certain embodiments, oligonucleotide sequences for amplification primers and/or detection probes specific for other CYP2C9 polymorphisms and/or polymorphisms of other cytochrome P540 genes *(e.g.,* CYP2D6) associated with adverse drug response are included an inventive kit. For example, at least 50%, at least 60%, at least 70%, at least 80%, at least 90% or more of the primers and/or probes in a kit comprise CYP2C9-specific oligonucleotide sequences described herein.

An inventive kit may further comprise instructions for using the amplification/ASPE reaction reagents and primer sets or primer/probe sets according to the present invention. Instructions for using the kit according to one or more methods of the invention may comprise instructions for processing the biological sample, extracting nucleic acid molecules from the sample, and/or performing the test; instructions for interpreting the results, including for using the results for diagnosis of an individual at risk for adverse drug response. For example, the kit may comprise an informational sheet or the like that describes how to interpret the results of the test and/or how to utilize the results of the test together with information regarding the existence or value of one or more classical risk factors in the individual. The kit may also comprise a notice in the form prescribed by a government agency *(e.g.,* FDA) regulating the manufacture, use or sale of pharmaceuticals of biological products. An identifier, *e.g.,* a bar code, radio frequency, ID tag, etc., may be present in or on the kit. The identifier can be used, *e.g.,* to uniquely identify the kit for purposes of quality control, inventory control, tracking, movement between workstations, etc. According to certain embodiments of the invention, the kits are manufactured in accordance with good manufacturing practices as required for FDA-approved diagnostic kits.

### Examples

The following examples describe some of the preferred modes of making and practicing the present invention. However, it should be understood that these examples are for illustrative purposes only and are not meant to limit the scope of the invention. Furthermore, unless the description in an Example is presented in the past tense, the text, like the rest of the specification, is not intended to suggest that experiments were actually performed or data were actually obtained.

### Example 1

An assay was carried out in a multiplex format using sets of primers and detection probes described in Table 1 (SEQ ID NOs. 1 to 4) and Table 2 (SEQ ID NOs. 5 through 12).

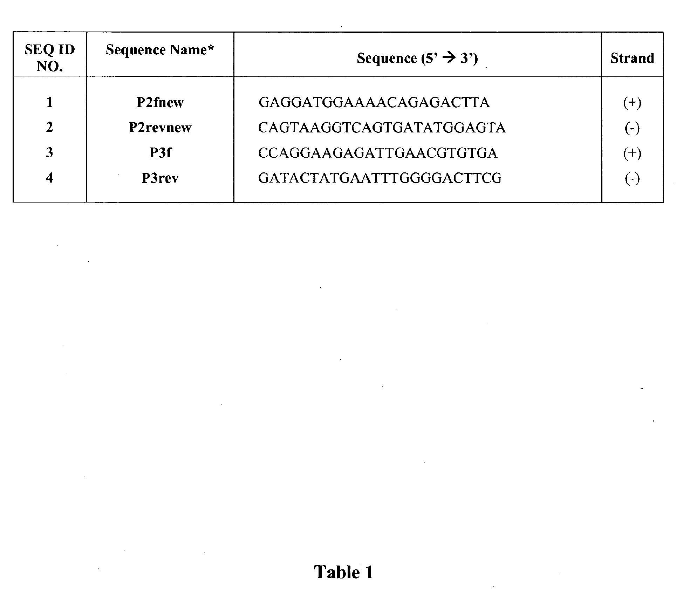

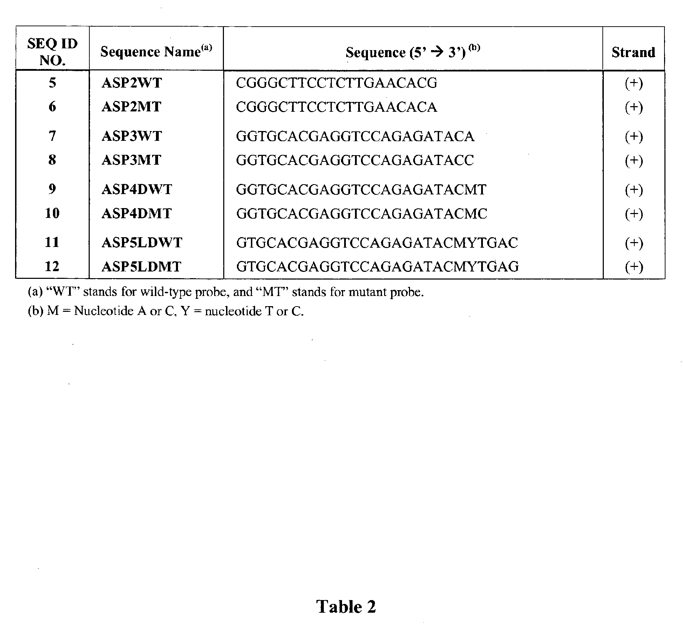

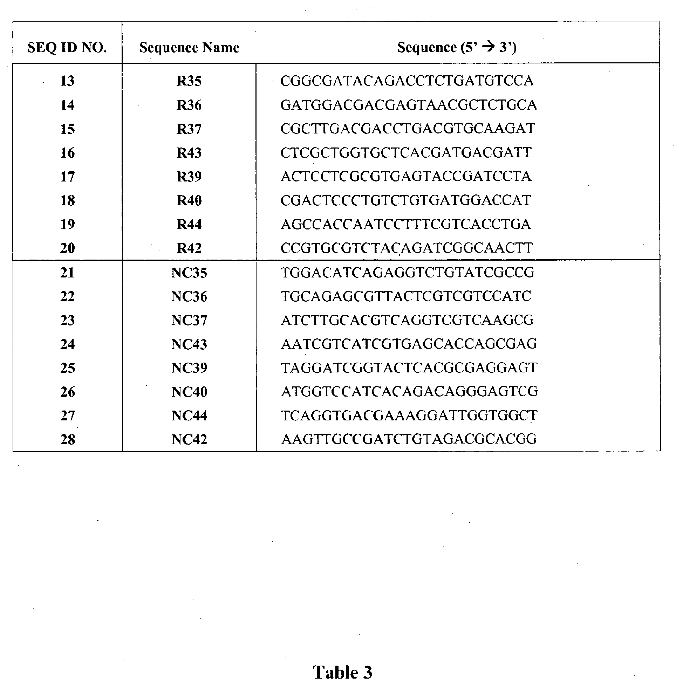

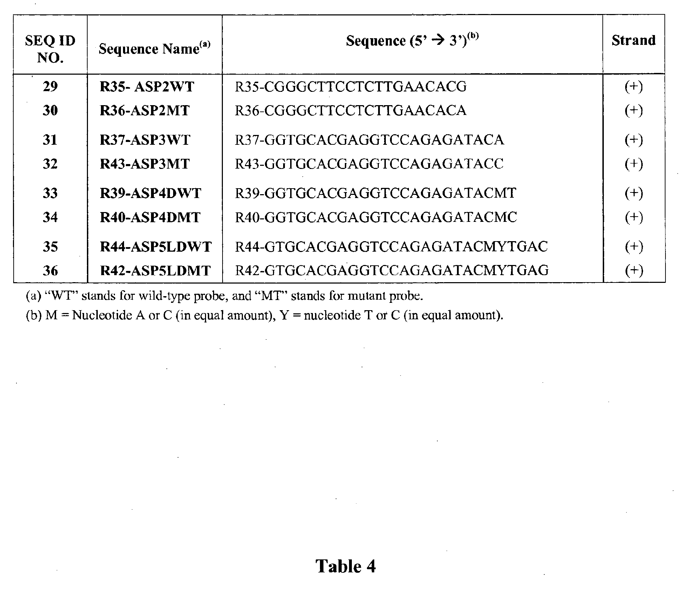

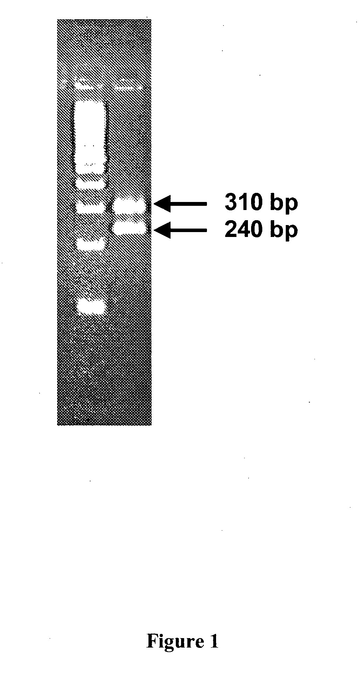

Amplification of genomic DNA obtained from an individual was performed using PCR. Amplification products obtained, Amplicon p2 and Amplicon p3, were found to have the correct sizes (*i.e.*, 310 bp and 240 bp, respectively) by agarose gel analysis (See Figure 1). PCR amplification was followed by Allele-Specific Primer Extension (ASPE) reaction. The allele-specific extension primers used were R35-ASP2WT, R36-ASP2MT, R37-ASP3WT, R43-ASP3MT, R39-ASP4DWT, R40-ASP4DMT, R44-ASP5LDWT, and R42-ASP5LDMT (*i.e*., inventive ASPE primers described in Table 4).

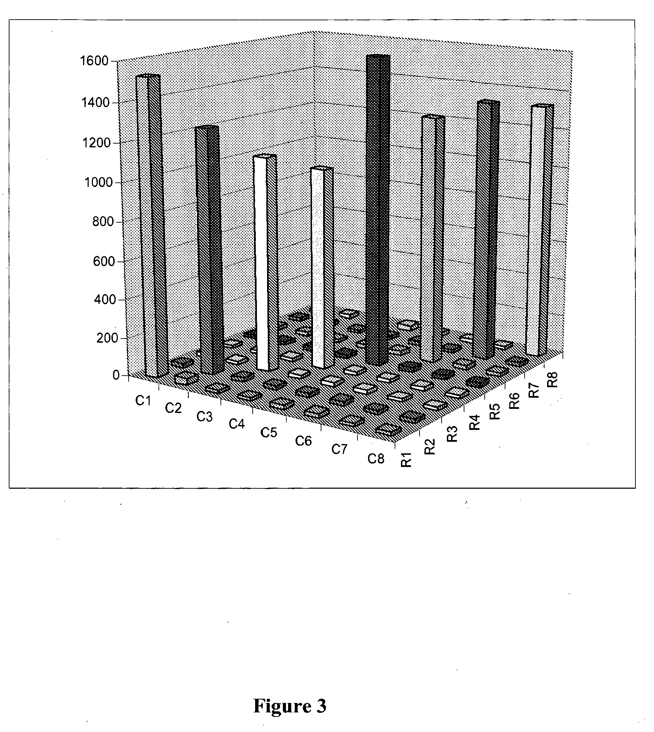

ASPE products were then captured onto Luminex beads coupled with orthogonal zipcode sequences of the invention (see Table 3). Performance (*i.e*., specificity) of the inventive tag and zipcode sequences was tested and the results are shown in Figure 3.

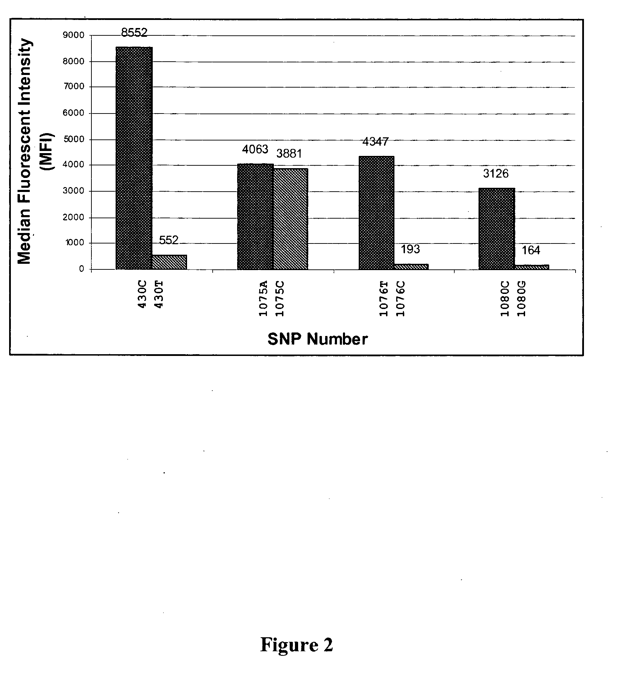

The final captured products were then detected using a Luminex-100 system. The genotyping results obtained, which show a good discrimination of all four SNPs, are presented in Figure 2. The individual tested was found to be heterozygous for 1075 A>C (*i.e*., SNP *3) and wild-type for the other polymorphisms.

### Example 2

A similar experiment was carried out using a panel of genomic DNA samples from seven patients that includes different available genotypes; and one negative control.

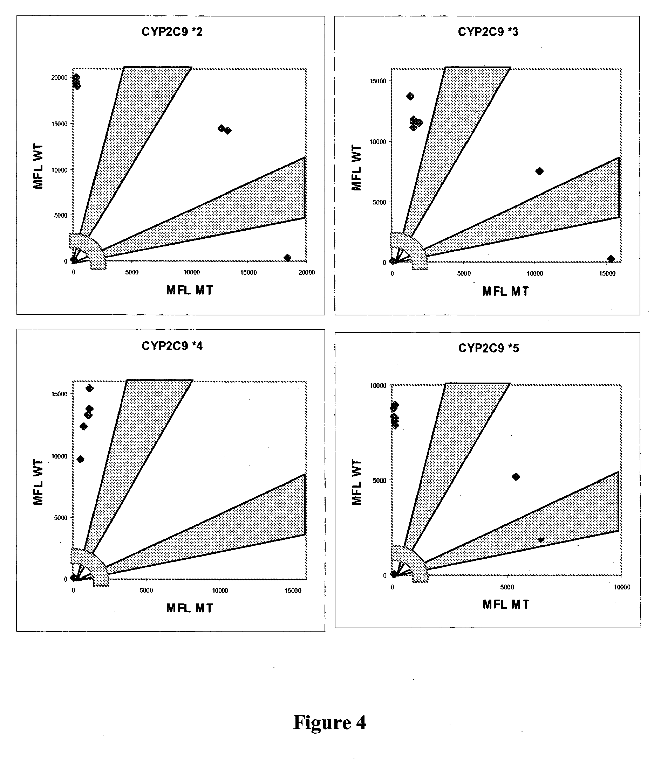

The results obtained in this experiment are presented in Figure 4 in one scattered plot per allele (*i.e*., CYP2C9 *2, CYP2C9 *3, CYP2C9 *4 and CYP2C9 *5). In these plots, values along the Y axis are MFL (mean fluorescence) recorded from the wild-type (WT) probes; values along the X axis are MFL recorded from the mutant probes. Grey zones are no-call regions (*i.e*., regions from which no conclusion can be drawn). Each small diamond represents one patient.

Data points that fall in the left hand-side region indicate homozygote wild-type genotypes; data points that fall in the middle region indicate heterozygote genotypes; and data points that fall in the right hand-side region indicate homozygote mutant genotypes. The data point at or near the origin corresponds to the negative control.

### Example 3

The ASPE products obtained as described in Example 1 were also detected using a planar waveguide platform. The ASPE reaction mixture was loaded on a PWG chip on which zipcode sequences of the present invention were spotted to specific locations for hybridization. The PWG chip was then washed and read using a PWG reader.

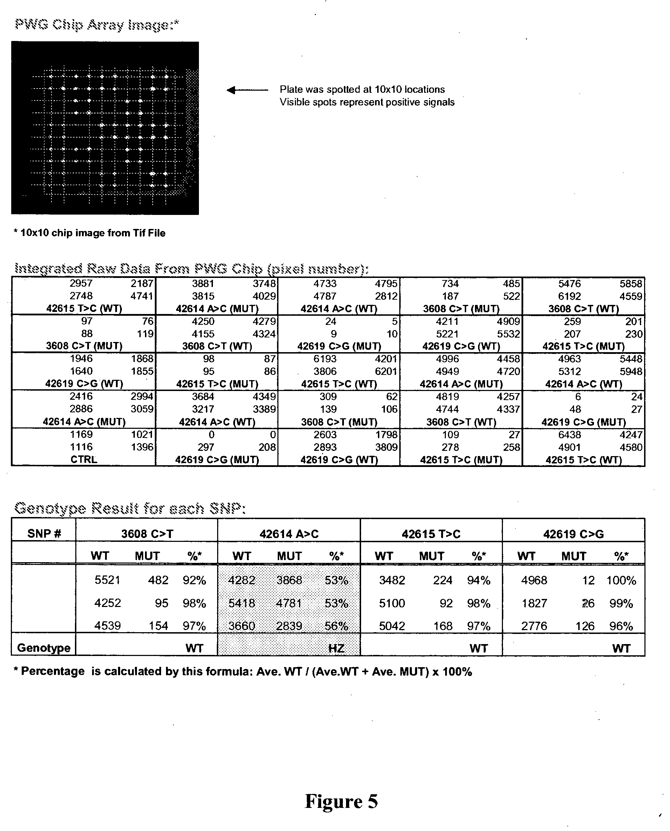

The results obtained, which are shown in Figure 5, are in complete concordance with the results obtained using the Luminex system. More specifically, the PWG analysis led to the conclusion that the individual tested is heterozygous for *3 and wild-type for the other polymorphisms.

### Other Embodiments

Other embodiments of the invention will be apparent to those skilled in the art from a consideration of the specification or practice of the invention disclosed herein. It is intended that the specification and examples be considered as exemplary only, with the true scope of the invention being indicated by the following claims.

### SEQUENCE LISTING

<110> Zheng, Minxue Ku, Lailing Shen, Lu-ping Wong, Carrie Bush-Donovan, Charlene
<120> Reagents and Methods for Detecting CYP2C9 Polymorphisms
<130> 2007P56002WOUS
<140> Not yet assigned
   <141> 2008-01-22
<150> PCT/US08/051613
   <151> 2008-01-22
<150> 60/881,740
   <151> 2007-01-22
<160> 36
<170> PatentIn version 3.5
<210> 1
   <211> 22
   <212> DNA
   <213> Homo sapiens
<400> 1
   gaggatggaa aacagagact ta 22
<210> 2
   <211> 24
   <212> DNA
   <213> Homo sapiens
<400> 2
   cagtaaggtc agtgatatgg agta 24
<210> 3
   <211> 23
   <212> DNA
   <213> Homo sapiens
<400> 3
   ccaggaagag attgaacgtg tga 23
<210> 4
   <211> 24
   <212> DNA
   <213> Homo sapiens
<400> 4
   gatactatga atttggggac ttcg 24
<210> 5
   <211> 20
   <212> DNA
   <213> Homo sapiens
<400> 5
   cgggcttcct cttgaacacg 20
<210> 6
   <211> 20
   <212> DNA
   <213> Homo sapiens
<400> 6
   cgggcttcct cttgaacaca 20
<210> 7
   <211> 23
   <212> DNA
   <213> Homo sapiens
<400> 7
   ggtgcacgag gtccagagat aca 23
<210> 8
   <211> 23
   <212> DNA
   <213> Homo sapiens
<400> 8
   ggtgcacgag gtccagagat acc 23
<210> 9
   <211> 24
   <212> DNA
   <213> Homo sapiens
<400> 9
   ggtgcacgag gtccagagat acmt 24
<210> 10
   <211> 24
   <212> DNA
   <213> Homo sapiens
<400> 10
   ggtgcacgag gtccagagat acmc 24
<210> 11
   <211> 27
   <212> DNA
   <213> Homo sapiens
<400> 11
   gtgcacgagg tccagagata cmytgac 27
<210> 12
   <211> 27
   <212> DNA
   <213> Homo sapiens
<400> 12
   gtgcacgagg tccagagata cmytgag 27
<210> 13
   <211> 25
   <212> DNA
   <213> Homo sapiens
<400> 13
   cggcgataca gacctctgat gtcca 25
<210> 14
   <211> 25
   <212> DNA
   <213> Homo sapiens
<400> 14
   gatggacgac gagtaacgct ctgca 25
<210> 15
   <211> 25
   <212> DNA
   <213> Homo sapiens
<400> 15
   cgcttgacga cctgacgtgc aagat 25
<210> 16
   <211> 25
   <212> DNA
   <213> Homo sapiens
<400> 16
   ctcgctggtg ctcacgatga cgatt 25
<210> 17
   <211> 25
   <212> DNA
   <213> Homo sapiens
<400> 17
   actcctcgcg tgagtaccga tccta 25
<210> 18
   <211> 25
   <212> DNA
   <213> Homo sapiens
<400> 18
   cgactccctg tctgtgatgg accat 25
<210> 19
   <211> 25
   <212> DNA
   <213> Homo sapiens
<400> 19
   agccaccaat cctttcgtca cctga 25
<210> 20
   <211> 25
   <212> DNA
   <213> Homo sapiens
<400> 20
   ccgtgcgtct acagatcggc aactt 25
<210> 21
   <211> 25
   <212> DNA
   <213> Homo sapiens
<400> 21
   tggacatcag aggtctgtat cgccg 25
<210> 22
   <211> 25
   <212> DNA
   <213> Homo sapiens
<400> 22
   tgcagagcgt tactcgtcgt ccatc 25
<210> 23
   <211> 25
   <212> DNA
   <213> Homo sapiens
<400> 23
   atcttgcacg tcaggtcgtc aagcg 25
<210> 24
   <211> 25
   <212> DNA
   <213> Homo sapiens
<400> 24
   aatcgtcatc gtgagcacca gcgag 25
<210> 25
   <211> 25
   <212> DNA
   <213> Homo sapiens
<400> 25
   taggatcggt actcacgcga ggagt 25
<210> 26
   <211> 25
   <212> DNA
   <213> Homo sapiens
<400> 26
   atggtccatc acagacaggg agtcg 25
<210> 27
   <211> 25
   <212> DNA
   <213> Homo sapiens
<400> 27
   tcaggtgacg aaaggattgg tggct 25
<210> 28
   <211> 25
   <212> DNA
   <213> Homo sapiens
<400> 28
   aagttgccga tctgtagacg cacgg 25
<210> 29
   <211> 20
   <212> DNA
   <213> Homo sapiens
<400> 29
   cgggcttcct cttgaacacg 20
<210> 30
   <211> 20
   <212> DNA
   <213> Homo sapiens
<400> 30
   cgggcttcct cttgaacaca 20
<210> 31
   <211> 23
   <212> DNA
   <213> Homo sapiens
<400> 31
   ggtgcacgag gtccagagat aca 23
<210> 32
   <211> 23
   <212> DNA
   <213> Homo sapiens
<400> 32
   ggtgcacgag gtccagagat acc 23
<210> 33
   <211> 24
   <212> DNA
   <213> Homo sapiens
<400> 33
   ggtgcacgag gtccagagat acmt 24
<210> 34
   <211> 24
   <212> DNA
   <213> Homo sapiens
<400> 34
   ggtgcacgag gtccagagat acmc 24
<210> 35
   <211> 27
   <212> DNA
   <213> Homo sapiens
<400> 35
   gtgcacgagg tccagagata cmytgac 27
<210> 36
   <211> 27
   <212> DNA
   <213> Homo sapiens
<400> 36
   gtgcacgagg tccagagata cmytgag 27

## Claims

1. A kit comprising a collection of primer pairs, wherein said primer pairs are suitable for use in a single-plex or multiplex PCR reaction that comprises human genomic DNA, said collection of primer pairs comprising:
a primer pair which, when used in the PCR reaction, generates an amplification product that encompasses nucleotides 258247 to 258556 of the CYP2C9 genomic sequence; and
a primer pair which, when used in the PCR reaction, generates an amplification product that encompasses nucleotides 297373 to 297612 of the CYP2C9 genomic sequence comprising the following primer pairs:
Primer Pair 1 comprising a forward primer comprising SEQ. ID NO. 1 and a reverse primer comprising SEQ ID NO. 2 and
Primer Pair 2 comprising a forward primer comprising SEQ. ID NO. 3 and a reverse primer comprising SEQ. ID NO. 4.
further comprising a collection of allele-specific extension probe pairs, wherein said probe pairs can distinguish between CYP2C9 alleles that differ at a polymorphic position when used in a primer extension reaction, wherein the first of said extension probes is complementary to a wild-type CYP2C9 allele at the polymorphic position and the second of said extension probes is complementary to a mutant CYP2C9 allele at the polymorphic position, wherein said polymorphic position is selected from the group consisting of nucleotide 430, nucleotide 1075, nucleotide 1076, and nucleotide 1080, , wherein the collection of allele-specific extension probe pairs comprises:
Probe Pair 1' comprising a wild-type probe comprising SEQ. ID NO. 29 and a mutant probe comprising SEQ ID NO. 30 and
at least one of:
Probe Pair 2'(*3) comprising a wild-type probe comprising SEQ. ID NO. 31 and a mutant probe comprising SEQ ID NO. 32
Probe Pair 2'(*4) comprising a wild-type probe comprising SEQ. ID NO. 33 and a mutant probe comprising SEQ ID NO. 34 and
Probe Pair 2'(*5) comprising a wild-type probe comprising SEQ. ID NO. 35 and a mutant probe comprising SEQ. ID NO. 36,
further comprising at least one zipcode sequence attached to a solid support, wherein the zipcode sequence comprises a sequence selected from the group consisting of: SEQ. ID NO. 21, SEQ. ID NO. 22, SEQ. ID NO. 23, SEQ. ID NO. 24, SEQ. ID NO. 25, SEQ. ID NO. 26, SEQ. ID NO. 27, and SEQ. ID NO. 28.

2. The kit of claim 1, wherein the collection of allele-specific extension probe pairs comprises:
Probe Pair 1 comprising a wild-type probe comprising SEQ ID NO. 5 and a mutant probe comprising SEQ ID NO. 6 and
at least one of:
Probe Pair 2(*3) comprising a wild-type probe comprising SEQ ID NO. 7 and a mutant probe comprising SEQ ID NO. 8
Probe Pair 2(*4) comprising a wild-type probe comprising SEQ ID NO. 9 and a mutant probe comprising SEQ ID NO. 10 and
Probe Pair 2(*5) comprising a wild-type probe comprising SEQ. ID NO. 11 and a mutant probe comprising SEQ. ID NO. 12.

3. The kit of claim 2, wherein the probes are attached to a solid support.

4. The kit of claim 2, wherein the probes are attached to microparticles.

5. The kit of claim 2, wherein the probes are attached to an array.

6. The kit of claim 1, wherein the zipcode sequence is attached to a microparticle.

7. The kit of claim 1, wherein the zipcode sequence is attached to an array.

## Patentansprüche

1. Kit, umfassend eine Sammlung von Primer-Paaren, wobei sich die Primer-Paare zur Verwendung in einer Single-plex- oder Multiplex-PCR-Reaktion eignen, die menschliche genomische DNA umfasst, wobei die Sammlung von Primer-Paaren Folgendes umfasst:
ein Primer-Paar, durch dessen Verwendung in der PCR-Reaktion ein Amplifikationsprodukt erzeugt wird, das Nukleotide 258247 bis 258556 der genomischen CYP2C9-Sequenz umfasst, und
ein Primer-Paar, durch dessen Verwendung in der PCR-Reaktion ein Amplifikationsprodukt erzeugt wird, das Nukleotide 297373 bis 297612 der genomischen CYP2C9-Sequenz umfasst, umfassend die folgenden Primer-Paare:
Primer Pair 1, umfassend einen SEQ. ID NO. 1 umfassenden Vorwärts-Primer und einen SEQ. ID NO. 2 umfassenden Rückwärts-Primer, und
Primer Pair 2, umfassend einen SEQ. ID NO. 3 umfassenden Vorwärts-Primer und einen SEQ. ID NO. 4 umfassenden Rückwärts-Primer,
ferner umfassend eine Sammlung allelspezifischer Verlängerungssondenpaare, wobei mit den Sondenpaaren zwischen CYP2C9-Allelen unterschieden werden kann, die sich an einer polymorphen Position unterscheiden, wenn sie in einer Primer-Verlängerungsreaktion eingesetzt werden, wobei die erste der Verlängerungssonden zu einem CYP2C9-Wildtyp-Allel an der polymorphen Position komplementär ist und die zweite der Verlängerungssonden zu einem CYP2C9-Mutanten-Allel an der polymorphen Position komplementär ist, wobei die polymorphe Position aus der aus Nukleotid 430, Nukleotid 1075, Nukleotid 1076 und Nukleotid 1080 bestehenden Gruppe ausgewählt ist, wobei die Sammlung allelspezifischer Verlängerungssondenpaare folgende Paare umfasst:
Probe Pair 1', umfassend eine SEQ ID NO. 29 umfassende Wildtyp-Sonde und eine SEQ ID NO. 30 umfassende Mutanten-Sonde, sowie
wenigstens eines aus:
Probe Pair 2'(*3), umfassend eine SEQ ID NO. 31 umfassende Wildtyp-Sonde und eine SEQ ID NO. 32 umfassende Mutanten-Sonde,
Probe Pair 2'(*4), umfassend eine SEQ ID NO. 33 umfassende Wildtyp-Sonde und eine SEQ ID NO. 34 umfassende Mutanten-Sonde, und
Probe Pair 2'(*5), umfassend eine SEQ ID NO. 35 umfassende Wildtyp-Sonde und eine SEQ ID NO. 36 umfassende Mutanten-Sonde,
ferner umfassend wenigstens eine an einem festen Träger gebundene Zipcode-Sequenz, wobei die Zipcode-Sequenz eine aus der aus: SEQ. ID NO. 21, SEQ. ID NO. 22, SEQ. ID NO. 23, SEQ. ID NO. 24, SEQ. ID NO. 25, SEQ. ID NO. 26, SEQ. ID NO. 27 und SEQ. ID NO. 28 bestehenden Gruppe ausgewählte Sequenz umfasst.

2. Kit nach Anspruch 1, wobei die Sammlung allelspezifischer Verlängerungssondenpaare folgende Paare umfasst:
Probe Pair 1, umfassend eine SEQ ID NO. 5 umfassende Wildtyp-Sonde und eine SEQ ID NO. 6 umfassende Mutanten-Sonde, sowie
wenigstens eines aus:
Probe Pair 2(*3), umfassend eine SEQ ID NO. 7 umfassende Wildtyp-Sonde und eine SEQ ID NO. 8 umfassende Mutanten-Sonde,
Probe Pair 2(*4), umfassend eine SEQ ID NO. 9 umfassende Wildtyp-Sonde und eine SEQ ID NO. 10 umfassende Mutanten-Sonde, und
Probe Pair 2(*5), umfassend eine SEQ ID NO. 11 umfassende Wildtyp-Sonde und eine SEQ ID NO. 12 umfassende Mutanten-Sonde.

3. Kit nach Anspruch 2, wobei die Sonden an einem festen Träger gebunden sind.

4. Kit nach Anspruch 2, wobei die Sonden an Mikropartikeln gebunden sind.

5. Kit nach Anspruch 2, wobei die Sonden auf einem Array gebunden sind.

6. Kit nach Anspruch 1, wobei die Zipcode-Sequenz an einem Mikropartikel gebunden ist.

7. Kit nach Anspruch 1, wobei die Zipcode-Sequenz auf einem Array gebunden ist.

## Revendications

1. Trousse comprenant une collection de paires d'amorces, les paires d'amorces étant propres à être utilisées dans une réaction PCR monoplexe ou multiplexe, qui comprend de l'ADN génomique humain, la collection de paires d'amorces comprenant :
une paire d'amorces qui, lorsqu'elle est utilisée dans la réaction PCR, produit un produit d'amplification, qui englobe des nucléotides 258247 à 258556 de la séquence génomique CYP2C9, et
une paire d'amorces qui, lorsqu'elle est utilisée dans la réaction PCR, produit un produit d'amplification, qui englobe des nucléotides 297373 à 297612 de la séquence génomique CYP2C9, comprenant les paires d'amorces suivantes :
paire d'amorces 1 comprenant une amorce sens comprenant l'identification de séquence N° 1 et une amorce antisens comprenant l'identification de séquence N° 2 et
paire d'amorces 2 comprenant une amorce sens comprenant l'identification de séquence N° 3 et une amorce antisens comprenant l'identification de séquence N° 4 comprenant, en outre, une collection de paires de sondes d'extension spécifiques à un allèle, les paires de sondes pouvant distinguer entre des allèles de CYP2C9 qui diffèrent en une position polymorphe lorsqu'elles sont utilisées dans une réaction d'extension à amorce, la première de ces sondes d'extension étant complémentaire d'une allèle de CYP2C9 de type sauvage en la position polymorphe et la deuxième de ces sondes d'extension étant complémentaire d'un allèle mutant de CYP2C9 en la position polymorphe, la position polymorphe étant choisie dans le groupe consistant en le nucléotide 430, le nucléotide 1075, le nucléotide 1076 et le nucléotide 1080, la collection de paires de sondes d'extension spécifiques à un allèle comprenant :
une paire de sondes 1' comprenant une sonde de type sauvage comprenant l'identification de séquence N° 29 et une sonde mutante comprenant l'identification de séquence N° 30 et
au moins l'une de :
une paire de sondes 2'(*3) comprenant une sonde de type sauvage, comprenant l'identification de séquence N° 31, et une sonde mutante, comprenant l'identification de séquence N° 32
une paire de sondes 2'(*4) comprenant une sonde de type sauvage, comprenant l'identification de séquence N° 33, et une sonde mutante, comprenant l'identification de séquence N° 34 et
une paire de sondes 2'(*5) comprenant une sonde de type sauvage, comprenant l'identification de séquence N° 35, et une sonde mutante, comprenant
l'identification de séquence N° 36,
comprenant, en outre, au moins une séquence de code zip fixée à un support solide, la séquence de code zip comprenant une séquence choisie dans le groupe consistant en : l'identification de séquence N° 21, l'identification de séquence N° 22, l'identification de séquence N° 23, l'identification de séquence N° 24, l'identification de séquence N° 25, l'identification de séquence N° 26, l'identification de séquence N° 27 et l'identification de séquence N° 28,

2. Trousse suivant la revendication 1, dans laquelle la collection de paires de sondes d'extension spécifiques à un allèle comprend :
une paire de sondes 1 comprenant une sonde de type sauvage comprenant l'identification de séquence N° 5 et une sonde mutante comprenant l'identification de séquence N° 6 et
au moins l'une de :
une paire de sondes 2(*3) comprenant une sonde de type sauvage, comprenant l'identification de séquence N° 7, et une sonde mutante, comprenant l'identification de séquence N° 8
une paire de sondes 2(*4) comprenant une sonde de type sauvage, comprenant l'identification de séquence N° 9, et une sonde mutante, comprenant l'identification de séquence N° 10 et
une paire de sondes 2(*5) comprenant une sonde de type sauvage, comprenant l'identification de séquence N° 11, et une sonde mutante, comprenant l'identification de séquence N° 12.

3. Trousse suivant la revendication 2, dans laquelle les sondes sont fixées à un support solide.

4. Trousse suivant la revendication 2, dans laquelle les sondes sont fixées à des microparticules.

5. Trousse suivant la revendication 2, dans laquelle les sondes sont fixées à un réseau.

6. Trousse suivant la revendication 1, dans laquelle la séquence de code zip est fixée à une microparticule.

7. Trousse suivant la revendication 1, dans laquelle la séquence de code zip est fixée à un réseau.
